(19) Europäisches Patentamt
European Patent Office
Office européen des brevets

(11) Publication number: 0 095 105 B1

(12) # EUROPEAN PATENT SPECIFICATION

(45) Date of publication of patent specification: 27.01.88

(21) Application number: 83104717.0

(22) Date of filing: 13.05.83

(51) Int. Cl.[4]: C 07 D 401/04, C 07 D 233/70 // C07D213/81, C07D213/82, C07D215/54

(54) **Process for the preparation of 2-(5,5-disubstituted-4-oxo-2-imidazolin-2-yl)-nicotinic acids, quinoline-3-carboxylic acids, and benzoic acids.**

(30) Priority: 25.05.82 US 381818

(43) Date of publication of application: 30.11.83 Bulletin 83/48

(45) Publication of the grant of the patent: 27.01.88 Bulletin 88/04

(84) Designated Contracting States: BE CH DE FR GB IT LI NL SE

(56) References cited:
EP-A-0 041 623

CHEMICAL ABSTRACTS, vol. 49, no. 7, 10th April 1955, columns 4633b-4634e, Columbus, Ohio (US); A.KJAER: "Cyclodehydration of acylated alpha-amino acid amides. I. Saturated amides"

THE CHEMISTRY OF HETEROCYCLIC COMPOUNDS, 1953, pp. 95-97, ed. Arnold Weissberger, Interscience Publishers, Inc., New York (US); K.Hofmann: "Imidazole and its derivatives, part I"

(73) Proprietor: AMERICAN CYANAMID COMPANY
1937 West Main Street P.O. Box 60
Stamford Connecticut 06904-0060 (US)

(72) Inventor: Barton, Jerry Michael
522 Dutch Neck Road
East Windsor New Jersey 08561 (US)
Inventor: Long, Don Wesley
19 Royal Oak Road
Trenton New Jersey 08638 (US)
Inventor: Lotts, Kenneth Dale
1515 Nottinghill Lane
Trenton New Jersey 08619 (US)

(74) Representative: Wächtershäuser, Günter, Dr.
Tal 29
D-8000 München 2 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 095 105 B1

Courier Press, Leamington Spa, England.

**Description**

Summary of the invention

The invention is a method for the preparation of formula (I), 2-(5,5-disubstituted-4-oxo(or thiono)-2-imidazolin-2-yl)nicotinic acids, 3-quinoline-carboxylic acids or benzoic acids having the structure:

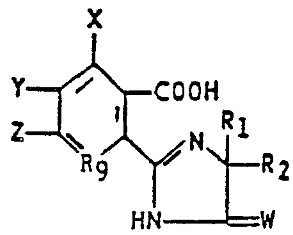

(IA)

by the base-catalyzed cyclization of a formula (XVa) 2-carbamoyl nicotinic acid or 2-carbamoyl 3-quinoline-carboxylic acid having the structure:

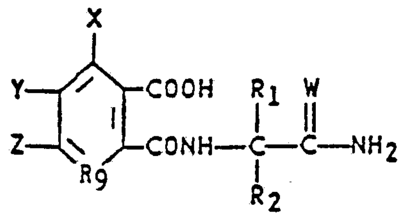

(XVa)

wherein $R_9$ is N or CH; $R_1$ is $C_1$—$C_4$ alkyl; $R_2$ is $C_1$—$C_4$ alkyl or $C_3$—$C_6$ cycloalkyl; and when $R_1$ and $R_2$ are taken together, along with the carbon to which they are attached, they may represent $C_3$—$C_6$ cycloalkyl optionally substituted with methyl, and when $R_1$ and $R_2$ are not the same, the optical isomers thereof; W is O or S; X is hydrogen, or $C_1$—$C_4$ alkyl, Y is hydrogen, halogen, $C_1$—$C_4$ alkyl, $C_1$—$C_4$ alkoxy, trifluoromethyl, trichloromethyl, difluoromethoxy, diloweralkylamino, $C_1$—$C_4$ alkylthio, nitro, phenyl, phenoxy, or phenyl or phenoxy substituted with one $C_1$—$C_4$ alkyl, $C_1$—$C_4$ alkoxy or halogen; Z represents hydrogen, $C_1$—$C_4$ alkyl, trifluoromethyl, trichloromethyl, phenyl or phenyl substituted with one $C_1$—$C_4$ alkyl, $C_1$—$C_4$ alkoxy or halogen; and, when taken together, Y and Z may form a ring in which YZ are represented by the structure: $-(CH_2)_n-$, where n is an integer selected from 3 to 5, provided that X is hydrogen; or YZ is

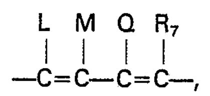

where L, M, Q and $R_7$ each represent hydrogen, halogen, $C_1$—$C_4$ alkyl, $C_1$—$C_4$ alkoxy, $C_1$—$C_4$ haloalkyl, difluoromethoxy, diloweralkylamino, $C_1$—$C_4$ alkylthio, nitro, phenyl, phenoxy, or mono-substituted phenyl or phenoxy where the substituent is one $C_1$—$C_4$ alkyl, $C_1$—$C_4$ alkoxy or halogen; with the proviso that only one of L, M, Q or $R_7$, may represent a substituent other than hydrogen, halogen, $C_1$—$C_4$ alkyl or $C_1$—$C_4$ alkoxy.

The base-catalyzed cyclization of this process involves reaction of a formula (XVa) carbamoyl nicotinic acid or carbamoyl 3-quinolinecarboxylic acid having the structure:

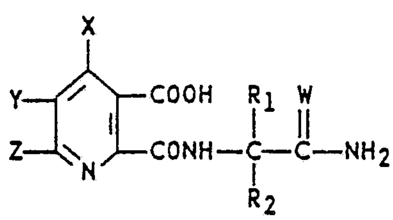

(XVa)

where X, Y, Z, W, $R_1$ and $R_2$ are as described above, with from 2 to 20 moles and preferably 2 to 6 moles of an aqueous or aqueous alcoholic sodium or potassium hydroxide, of 10% or greater concentration on a weight basis, per mole of formula (XVa) acid, at a temperature between 25° and 110°C, i.e., reflux temperature. Thereafter, the reaction mixture is acidified to a pH between 2 and 4 with a strong mineral acid to give, in high yield and purity, the 2-(5,5-disubstituted-4-oxo(or thiono)-2-imidazolin-2-yl)nicotinic acid or 3-quinolinecarboxylic acid having the structure of formula (IA):

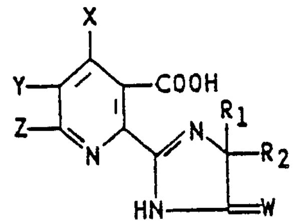

(IA)

wherein X, Y, Z, W, $R_1$ and $R_2$ are as described above.

Advantageously, the process of the invention is also effective for preparing the formula (IA) 2-(5,5-disubstituted-4-oxo(or thiono)-2-imidazolin-2-yl)-nicotinic acids and 3-quinolinecarboxylic acids from isomer mixtures of the formula (XVa) carbamoyl nicotinic acid or carbamoyl 3-quinolinecarboxylic acid and the formula (XVb) carbamoyl picolinic acid or carbamoyl quinaldic acid. In accordance with the process of the invention, the isomeric mixture containing the formula (XVa) carbamoyl nicotinic acid or quinolinic acid and the formula (XVb) carbamoyl picolinic or quinaldic acid is heated to a temperature between 25 and 110°C (i.e., reflux temperature) with about 2 to 20 molar equivalents of aqueous or aqueous alcoholic ($C_1$—$C_4$-alcohol) sodium or potassium hydroxide, and preferably 2 to 6 molar equivalents of said base of greater than 10% concentration on a weight basis under a blanket of inert gas such as nitrogen or argon. The mixture is then cooled to about 25°C and acidified to a pH between 2 and 4 with a strong mineral acid such as hydrochloric acid or sulfuric acid to give the herbicidally effective formula (IA) product in greater than 95% yields. If the formula (XVa) product is insoluble in water, it will be precipitated and can be recovered by filtration. If the product is soluble in water, the mixture can be extracted with an organic solvent such as ether or methylene chloride and the extract concentrated to provide the herbicidally effective 2-(5,5-disubstituted-4-oxo(or thiono)-2-imidazolin-2-yl)nicotinic or 3-quinoline-carboxylic acid encompassed by formula (IA). A reaction reported by A. Kjaer, *Acta. Chemica. Scand 7,* 889, (1953) utilizing dilute 1.0N (4%) aqueous sodium hydroxide gives significantly lower yields (10 to 15% lower).

The Chemistry of Heterocyclic Compounds (1953) on page 95 discloses a process for the preparation of 2-phenyl-5,5-dimethyl-4-(5H)-imidazolinone in hot dilute sodium hydroxide. The product thus formed exhibits poor chemical stability and fragments upon treatment with strong acids and alkalies.

The process of the invention also relates to a method for the preparation of the herbicidally effective formula (IA) 2-(5,5-disubstituted-4-oxo(or thiono)-2-imidazolin-2-yl)nicotinic acids, 3-quinolinecarboxylic acids, and benzoic acids by reaction of an appropriately substituted formula (XVI) anhydride with an aminocarboxyamide or aminothiocarboxamide depicted by formula (XIIIa) to yield an isomeric mixture of the formula (XVa) and formula (XVb) carbamoyl nicotinic, quinoline-carboxylic, or benzoic acid and the carbamoyl picolinic or quinaldic acid. This reaction is carried out, preferably using equivalent amounts of the formula (XVI) anhydride and the formula (XIIIa) carboxamide or thiocarboxamide, in the presence of an inert organic solvent such as low-boiling ether (diethyl ether, tetrahydrofuran or dimethoxyethane), acetonitrile, ethyl acetate or a halogenated hydrocarbon such as methylene chloride. The reaction is generally conducted at a temperature between 20° and 80°C and preferably at 30° to 60°C under a blanket of inert gas such as nitrogen or argon. This reaction yields the isomeric mixture of the formula (XVa) carbamoyl nicotinic, 3-quinolinecarboxylic, or benzoic acids and the formula (XVb) carbamoyl picolinic or quinaldic acids. The isomeric mixture may then be treated as described above to recover the herbicidally effective formula (IA) 2-(5,5-disubstituted-4-oxo(or thiono)-2-imidazolin-2-yl)nicotinic acids, 3-quinolinecarboxylic, or benzoic acids.

The above described reactions are graphically illustrated in Flow Diagram I.

The herbicidal 2-(5,5-disubstituted-4-oxo-2-imidazolin-2-yl)nicotinic acids and quinoline-3-carboxylic acids prepared according to the process of this invention are described and exemplified in the European Patent Application Publication number 0041623 published December 16, 1981 Bulletin 81/50, Application number 81103638.3 of Marinus Los. The herbicidal benzoic acids are described and exemplified in U.S. Patent 4,188,487 (1980) of Marinus Los.

FLOW DIAGRAM I

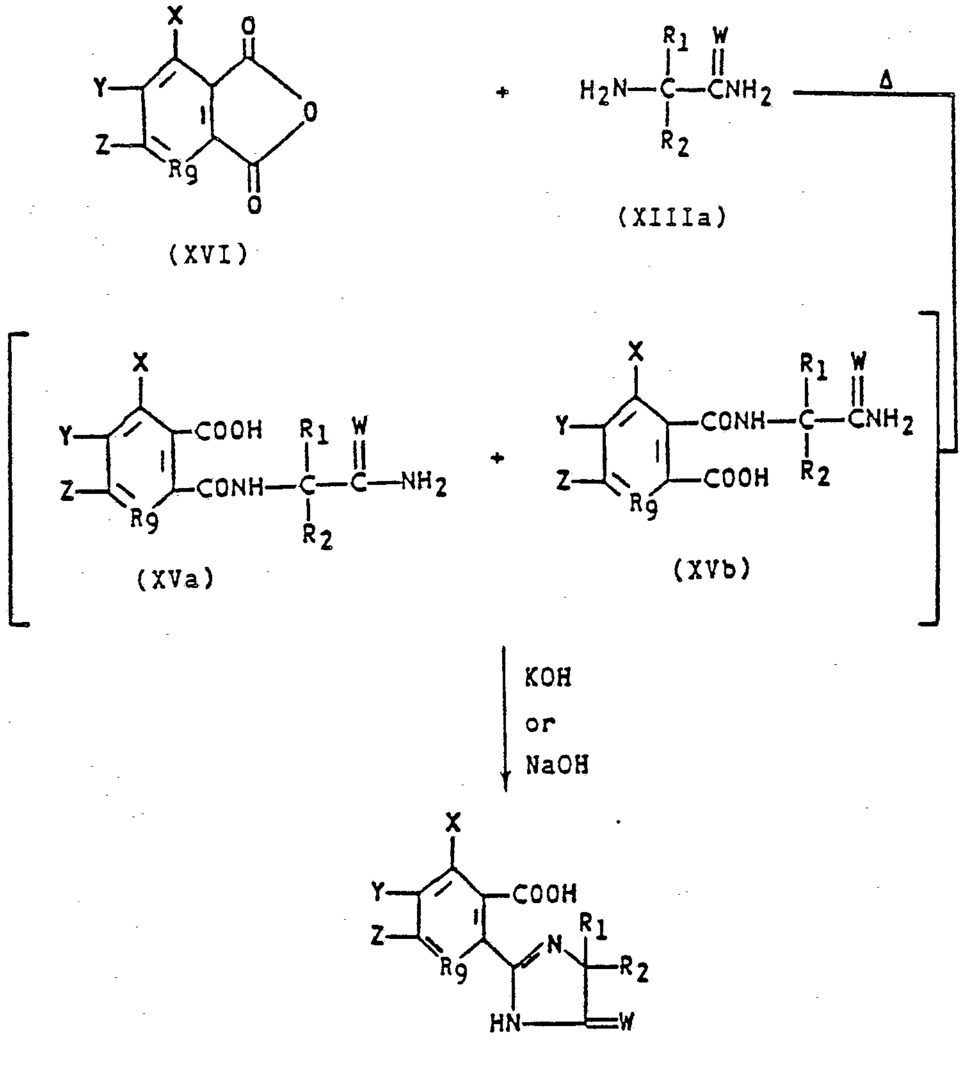


(XVI) (XIIIa)

(XVa) (XVb)

(IA)

wherein X, Y, Z, W, $R_1$ and $R_2$ are as described above and $R_9$ is N or CH.

Conveniently, the method of the present invention is also suitable for the preparation of the imidazolinyl-quinolinic, nicotinic, and benzoic acid herbicides directly from nitriles by hydrolysis of the nitrile in situ and with a strong base and hydrogen peroxide in a single operation as graphically illustrated below:

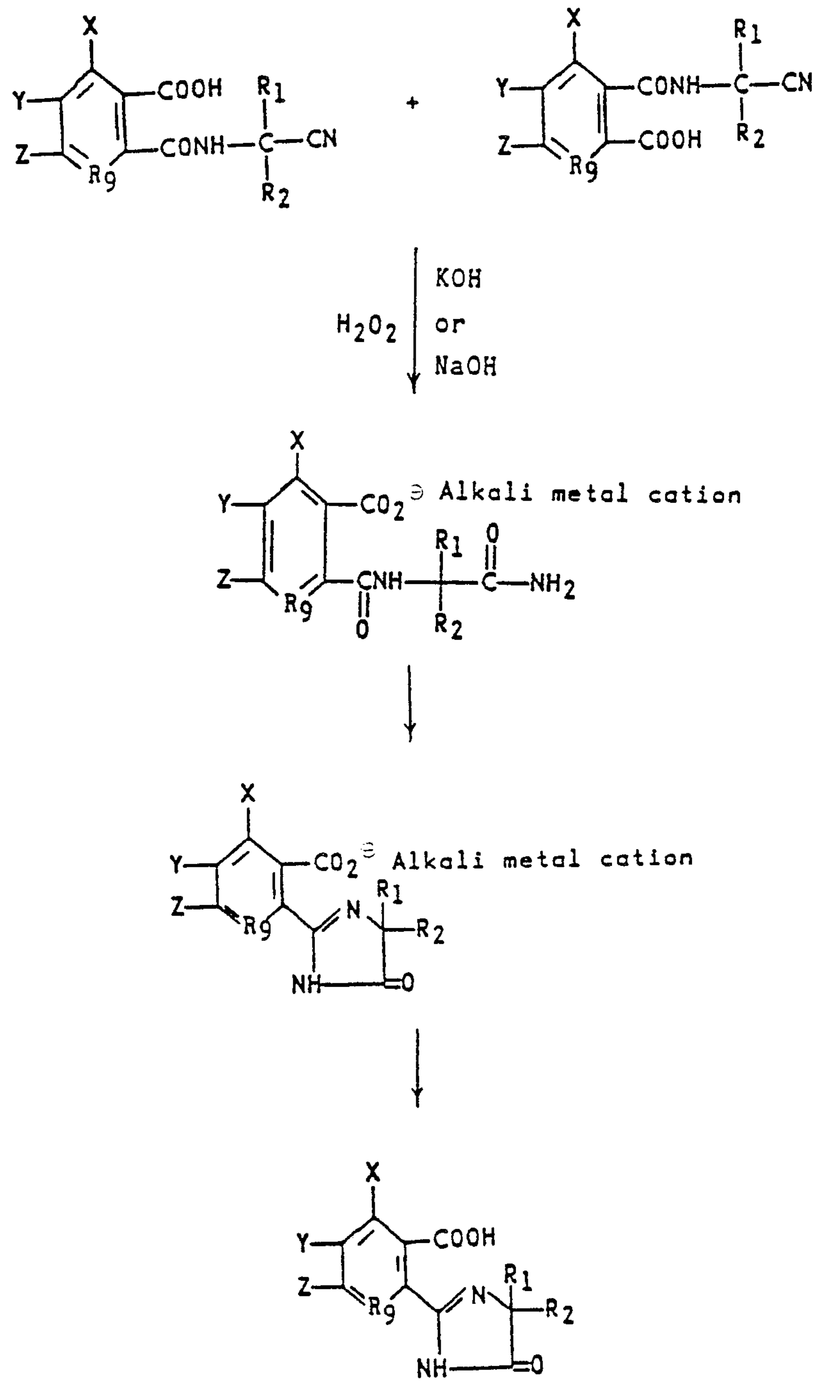


(IB)

wherein $R_9$, X, Y, Z, $R_1$ and $R_2$ are as described above.

The reactions may be carried out at 0—100°C in water, a mixture of water and water miscible solvents such as $C_1$—$C_4$ alcohol or a mixture of water and water immiscible solvents such as dichloromethane, 1,2-dichloroethane, chlorobenzene, toluene, xylenes and ethyl ether. Reactions in water at 60—100°C are preferred as cyclization to the desired imidazolinyl products is more rapid at elevated temperatures, and disposal or recovery of organic solvents is not necessary.

The use of aqueous 30—90% hydrogen peroxide with alkali metal hydroxides serves to speed the conversion of the acid amidonitriles to the alkali metal cation salts of the acid diamides and results in less by-product formation. The peroxide may be employed in a molar ratio of 0 to 10 moles, preferably 2 to 5 moles, per mole of acid amidonitrile.

Both aqueous and aqueous alcoholic sodium or potassium hydroxide may be employed in molar ratios of 2 to 10 moles, preferably 2 to 6 moles of 10% or greater concentration on a weight basis, per mole of acid amidonitrile.

The product imidazolinyl-acids are amphoteric and are capable of acting either as acids or bases. Thus, they may be isolated as the alkali metal cation salts, as the free acids, and when treated with strong acids such as hydrochloric, sulfuric and hydrobromic acids, as acid addition salts.

The acid amidonitriles suitable for use as starting materials are conveniently prepared by first reacting methyl isopropyl ketone with hydrogen cyanide in aqueous ammonium hydroxide to obtain 2-amino-2,3-dimethylbutyronitrile. This compound is then reacted with the appropriate anhydride to give the acid amidonitriles. This preparation is described in United States Patent 4,017,510.

The formula (IA) 2-(5,5-disubstituted-4-oxo-(or thiono)-2-imidazolin-2-yl)nicotinic acids, 3-quinolinecarboxylic acids, and benzoic acids, prepared by the process of the present invention, are highly effective herbicidal agents useful for the control of a wide variety of monocotyledonous and dicotyledonous plants.

They are also useful as aquatic herbicides and are unique in their effectiveness in controlling plants when applied to the foliage thereof, or to the soil, or water containing seeds, or other propagating organs of the plants such as tubers, rhizomes or stolons, at rates of from about 0.025 to 8.0 kg/ha, and preferably at rates from about 0.032 to 4.0 kg/ha.

In order to facilitate a further understanding of the invention, the following examples are presented primarily for the purpose of illustrating certain more specific details thereof. The invention is not to be deemed limited thereby except as defined in the claims.

Example 1

Preparation of 2-(5-isopropyl-5-methyl-4-oxo-2-imidazolin-2-yl)nicotinic acid

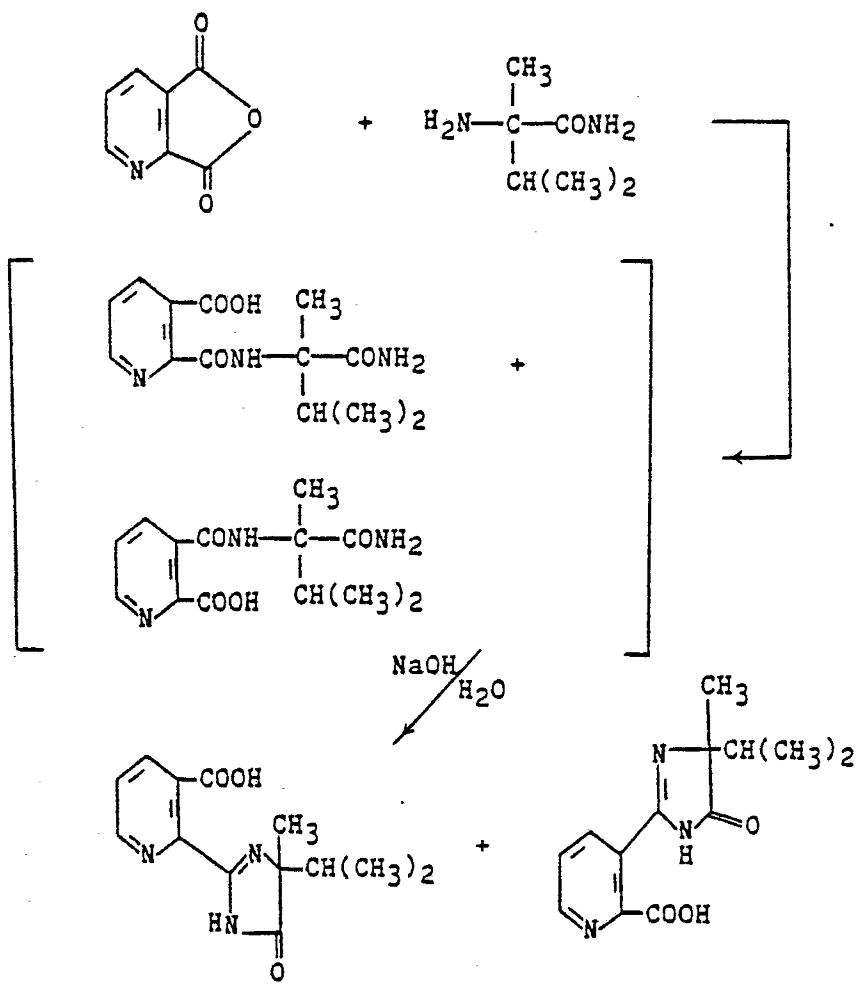


To a stirred suspension of 2,3-pyridinecarboxylic anhydride (30 g) in 150 mL of acetonitrile is added a solution of 2-amino-2,3-dimethylbutyramide (28 g) in 140 mL of acetonitrile at 25° to 30°C. The mixture is stirred for 2 hours. The solvent is removed at 50°C and reduced pressure. The residual gum is dissolved in 230 mL of 2.6 N sodium hydroxide and heated to 80°C for 1.5 hours.

The mixture is cooled to 25°C and acidified to a pH of 3 with 65 mL of 37% hydrochloric acid. The resulting solution is extracted with two 200 mL portions of methylene chloride. The extracts are concentrated to a residue of 33 g of the desired product, mp 160—165°C.

After standing overnight, the aqueous layer deposits 3.8 g of the picolinic acid isomer, mp 155—157°C (dec.).

Example 2

Preparation of 2-(4-oxo-1,3-diazaspiro[4.5]dec-2-en-2-yl)nicotinic acid

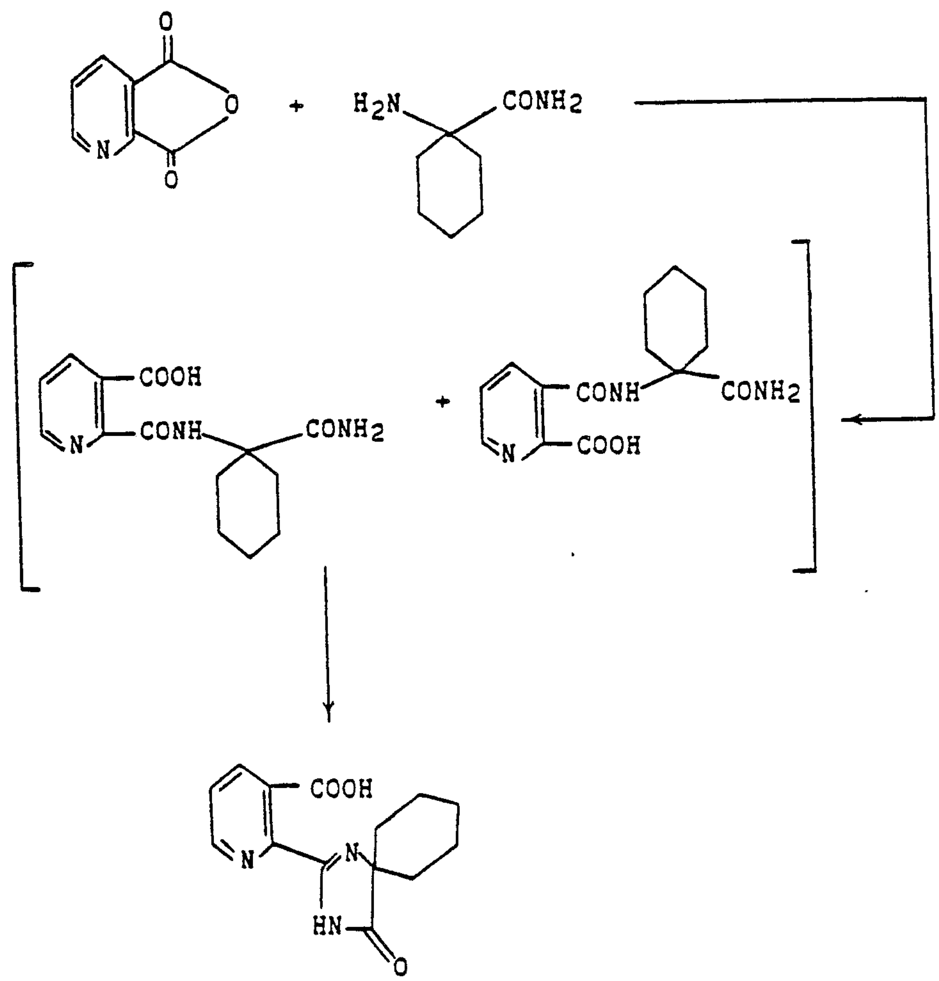

To a stirred solution of 7.1 g of 1-aminocyclohexanecarboxamide in 60 mL of methylene chloride is added 7.5 g of 2,3-pyridinedicarboxylic anhydride. The mixture becomes warm and forms a solution. Stirring is continued for two hours as a colorless solid precipitates. The mixture of monoacids is collected, 12.0 g, mp 168—178°C (dec.).

This material is dissolved in 45 mL of 2.7 N sodium hydroxide and heated for one hour at 80—85°C. It is then cooled, acidified with 10.3 mL of 37% hydrochloric acid, and extracted with two 25 mL portions of methylene chloride. The extracts are concentrated to give 7.5 g of the desired product which is recrystallized from aqueous methanol to give 2-(4-oxo-1,3-diazaspiro[4.5]dec-2-en-2-yl)nicotinic acid, mp 186—189°C.

Example 3

Preparation of 6-isopropyl-2-(5-isopropyl-5-methyl-4-oxo-2-imidazolin-2-yl)nicotinic acid

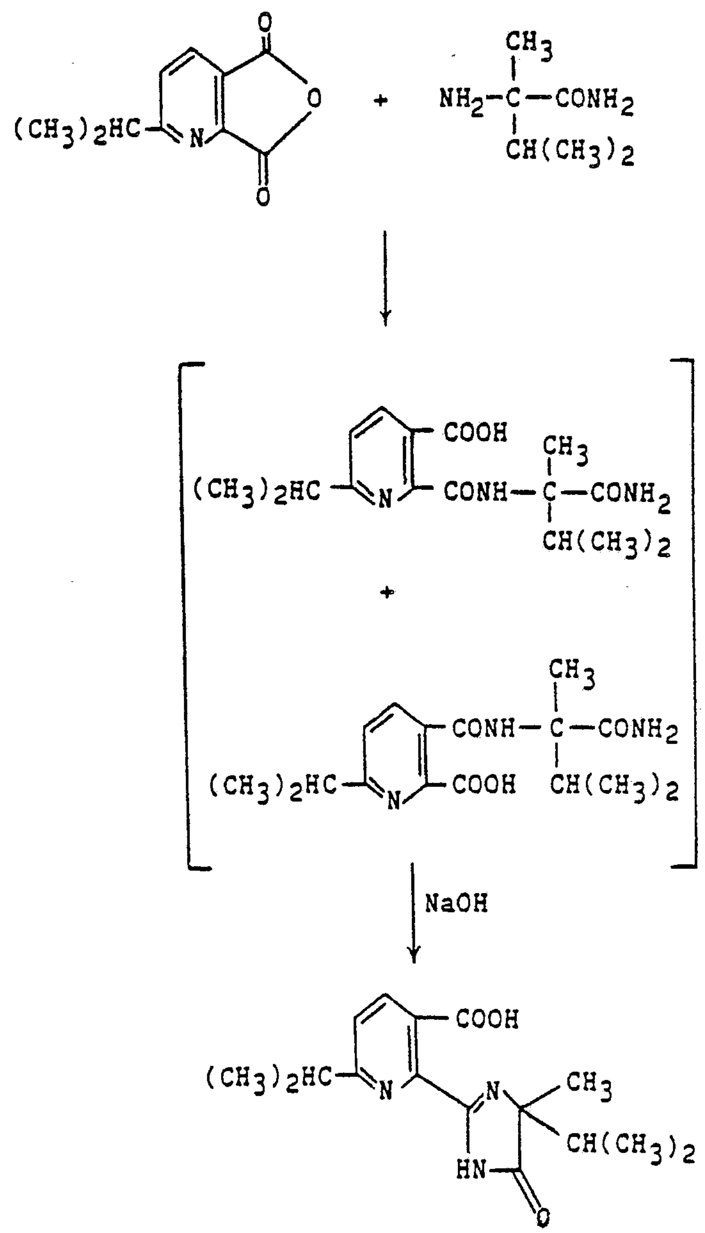


To a stirred solution of the anhydride (15.1 g) in 125 mL THF under nitrogen is added 11.4 g of 2-amino-2,3-dimethylbutyramide. The mixture is stirred overnight. The solvent is removed *in vacuo*, and the resulting oil (consisting of a mixture of the isomeric pyridine monoacid products) dissolved in 66 mL of 6N NaOH. This solution is heated at 70°C under nitrogen for three and one-half hours, then cooled and the pH of the solution adjusted to 9 with 6N $H_2SO_4$. The mixture is extracted with ether twice, and the organic extracts discarded. The pH of the aqueous phase is adjusted to 3 with 6N $H_2SO_4$. The resulting precipitate is removed by filtration, washed with water and dried to give 13.25 g of desired product. A sample is recrystallized from methylene chloride-hexane followed by ether-hexane to give an analytically pure sample of 6-isopropyl-2-(5-isopropyl-5-methyl-4-oxo-2-imidazolin-2-yl)nicotinic acid, mp 131—133.5°C.

By using essentially the same procedure, but substituting the appropriate substituted pyridine-2,3-dicarboxylic acid anhydride and also substituting, if necessary, the optically active 2-amino-2,3-dimethylbutyramide or the 2-amino-2,3-dimethylthiobutyramide for 2-amino-2,3-dimethylbutyramide, the following nicotinic acids were prepared.

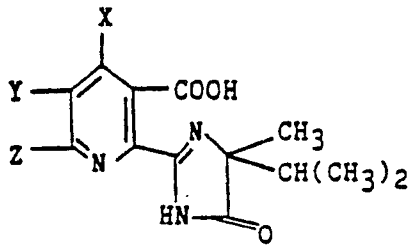

| X | Y | Z | mp°C |
|---|---|---|---|
| H | H | $CH_3$ | 145—146.5 |
| H | H | $CF_3$ | 133—142 |
| H | H | H | 128—131<br>$[\alpha]_D^{25}$ = 18.37°<br>(c=0.0902 g/ml THF) |
| H | H | *n*-$C_3H_7$ | 148.5—150.5 |
| H | H | 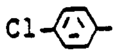 | 247—249 |
| H | H | 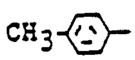 | 215.5—218.5 |
| H | H | 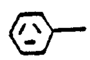 | 252—254 |
| H | H | $C_2H_5$ | 118—122 |
| H | $CH_3$ | $CH_3$ | 172—180 |
| H | $-(CH_2)_3-$ | | 160—164 |
| H | H | H | 170—172.5 |
| H | $-(CH_2)_4-$ | | 162—165 |
| H | $-(CH_2)_5-$ | | 141—148 |

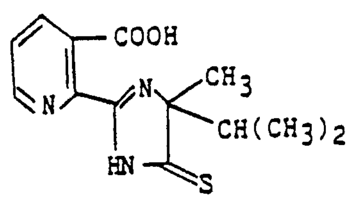

mp 182—184°

Example 4

Preparation of 2-(5-isopropyl-5-methyl-4-oxo-2-imidazolin-2-yl)-3-quinolinecarboxylic acid

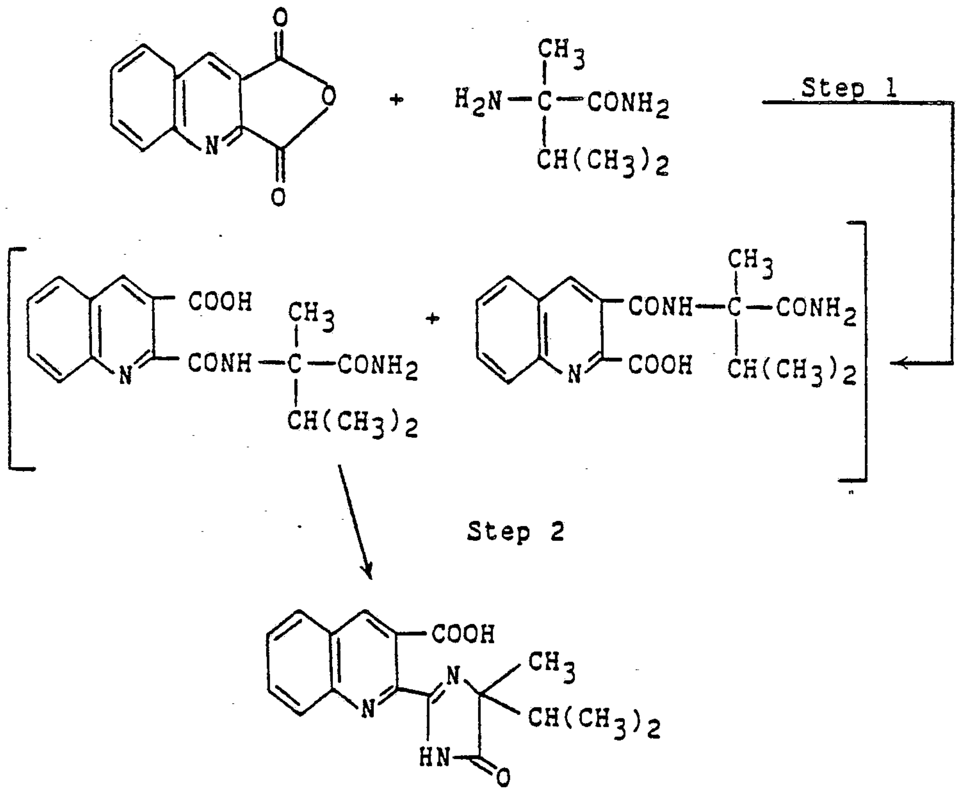


To a stirred solution of 2-amino-2,3-dimethylbutyramide (40 g) in 500 mL of acetonitrile is added 60 g of 2,3-quinolinedicarboxylic acid anhydride in portions during about 45 minutes. The mixture is heated to 50—60°C for two hours, cooled to room temperature and filtered to give 73.7 g of the mixture of carbamoyl quinolinecarboxylic acids.

This solid is dissolved in 435 mL of 1.5 N sodium hydroxide and heated for two hours at 80—85°C. The solution is cooled and acidified with 57 mL of 37% hydrochloric acid. The desired product is removed by filtration and dried. The solid is recrystallized from methanol to give 49 g of 2-(5-isopropyl-5-methyl-4-oxo-2-imidazolin-2-yl)-3-quinolinecarboxylic acid mp 240—242°C.

Following step 1 of the above procedure yields the following 2-carbamoyl-3-quinolinecarboxylic acids having the structure:

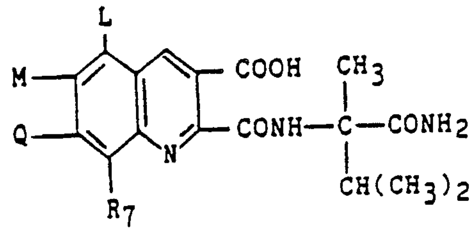

wherein L, M, Q and $R_7$ are as reported below.

| L | M | Q | $R_7$ | mp°C |
|---|---|---|---|---|
| H | H | H | H | 172.5—173.5 |
| H | H | H | H | 183—185<br>$[\alpha_D] = -90.5°$ in $CH_2Cl_2$ |
| H | $OC_2H_5$ | H | H | — |
| H | $NO_2$ | H | H | 225—227 |
| H | H | H | $OCH_3$ | Foam |
| H | $CF_3$ | H | H | 222—224 |
| H | CN | H | H | — |
| H | $C_6H_5$ | H | H | 189.5—192 |
| H | H | $CH_3$ | $CH_3$ | 246—250 |
| H | $OCH_3$ | H | H | — |
| H | $CH_3$ | $CH_3$ | H | — |
| H | $C_2H_5$ | H | H | 198—199 |
| H | $C_5H_9$ | H | H | 163—164 |
| H | Br | H | H | — |
| $OCH_3$ | H | H | $OCH_3$ | 209—209.5 |
| H | $SCH_3$ | H | H | — |
| H | $OC_6H_5$ | H | H | 189—190 |
| H | $OCF_2H$ | H | H | 194—196 |
| H | H | $OC_2H_5$ | H | — |

Following step 2 of the above procedure, i.e., the base-catalyzed cyclization of a carbamoyl-3-quinolinecarboxylic acid yields the 2-(5-isopropyl-5-methyl-4-oxo-2-imidazolin-2-yl)-3-quinolinecarboxylic acids having the structure:

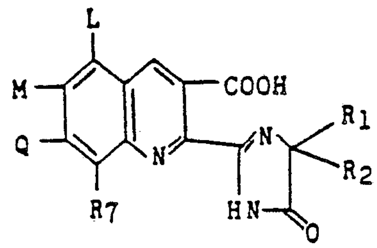

wherein L, M, Q and $R_7$ are as reported below.

| L | M | Q | $R_7$ | mp°C |
|---|---|---|---|---|
| H | H | H | H | 241—244 |
| H | H | H | H | 228—236.5 $[\alpha]_D^{25} = +28.3°$ (c=0.0105g/mL$CH_2Cl_2$) |
| H | $OC_2H_5$ | H | H | 206—208 |
| H | $NO_2$ | H | H | 241.5—242 |
| H | H | H | $OCH_3$ | 258—261 |
| H | $CF_3$ | H | H | 215—218 |
| H | CN | H | H | — |
| H | $C_6H_5$ | H | H | 209.5—212 |
| H | H | $CH_3$ | $CH_3$ | 280 |
| H | $OCH_3$ | H | H | 203.5—205 |
| H | $CH_3$ | $CH_3$ | H | 238—240 |
| H | $C_2H_5$ | H | H | 179—180.5 |
| H | $C_4H_9$ | H | H | 149—150.5 |
| H | Br | H | H | 215—225 |
| $OCH_3$ | H | H | $OCH_3$ | 249—250 |
| H | $SCH_3$ | H | H | 264—265 |
| H | H | $OC_2H_5$ | H | — |
| H | $OC_6H_5$ | H | H | 223 |
| H | $OCF_2H$ | H | H | 208—209 |

Example 5

Preparation of 2-(5-Isopropyl-5-methyl-4-oxo-2-imidazolin-2-yl)benzoic acid

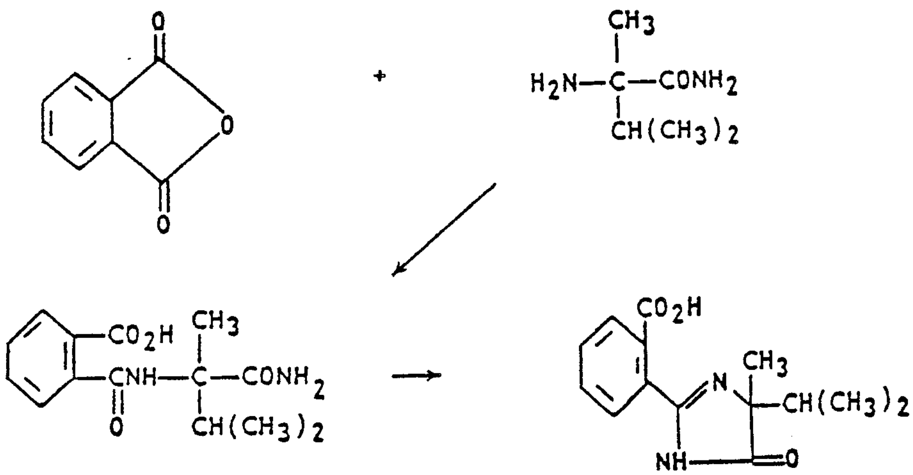

To a stirred solution of 13.4 g of 2-amino-2,3-dimethylbutyramide in 200 mL of methylene chloride is added 13.4 g of phthalic anhydride. The mixture is warmed to reflux for a few minutes and then left to cool and stir overnight. The clear solution is then stirred with 160 mL of 0.8 N sodium hydroxide for 15 minutes. The aqueous layer is separated and treated with another 12.1 g of 50% aqueous sodium hydroxide and the alkaline solution is heated to 75°C for 2.5 hours.

The solution is then cooled to 25°C and neutralized with 23 mL of 37% aqueous hydrochloric acid. Colorless product separates and is collected and dried to give 13.8 g, mp 158—162°C. The filtrate is concentrated to a volume of 50 mL at reduced pressure to give another 10.0 g of product melting at 150—170°C. Total yield of crude product is 23.8 g of 91.5%.

Example 6

Preparation of 2-(5-isopropyl-5-methyl-4-oxo-2-imidazolin-2-yl)-*p*-toluic acid and 6-(5-isopropyl-5-methyl-4-oxo-imidazolin-2-yl-*m*-toluic acid

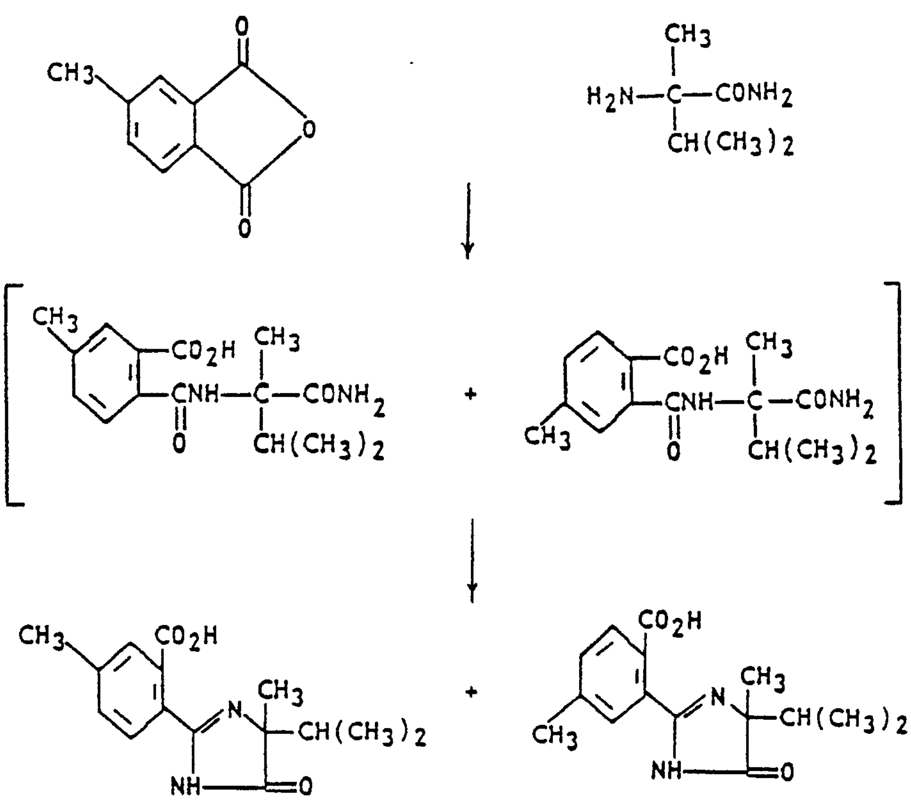

To a solution of 14.4 g of 2-amino-2,3-dimethylbutyramide in 75 mL of acetonitrile is added 16.2 g of 4-methylphathalic anhydride. The solution is warmed to 60°C for two hours and then concentrated at reduced pressure to a residue of 31.3 g. The residue is dissolved in 80 mL of 3N sodium hydroxide and warmed to 80—85°C for three hours. It is then cooled to 25°C and neutralized with 23.5 mL of 37% aqueous hydrochloric acid. Near the end of the neutralization, 100 mL of methylene chloride is added to dissolve the gummy product which is separating. The layers are separated and the aqueous layer is extracted with an additional 65 mL of methylene chloride. The combined methylene chloride layers are concentrated to a residual gum weighing 28.8 g which is shown by quantitative high performance liquid chromatography assay to contain 23.9 g of the two desired products. The yield is therefore 87%.

Example 7

Preparation of 2-(5-isopropyl-5-methyl-4-oxo-2-imidazolinyl-2-yl)-*p*-toluic acid and 6-(5-isopropyl-5-methyl-4-oxo-2-imidazolin-2-yl)-*m*-toluic acid

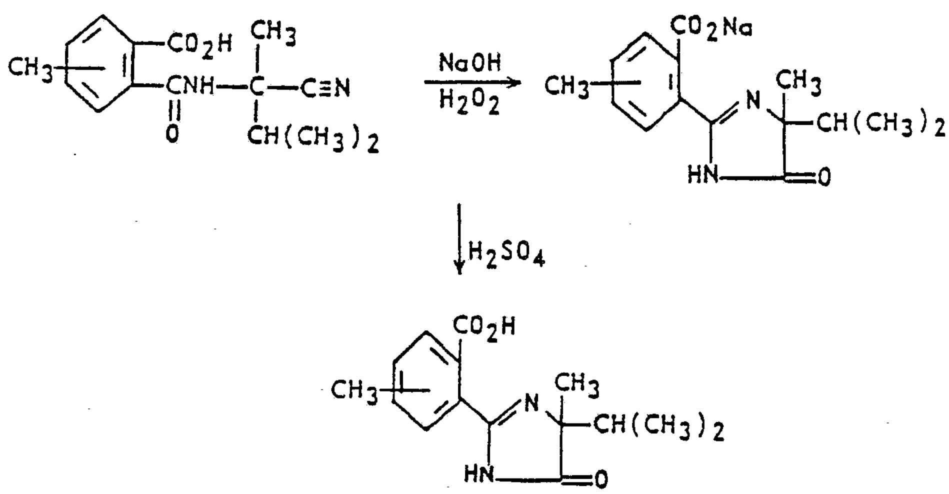


To 32.0 g (0.116 mol) of N-(1-cyano-1,2-dimethylpropyl)-4(and 5)-methyl phthalamic acid in 80 mL water is added 30.0 g (0.375 mol) of 50% sodium hydroxide. External cooling is applied to hold the temperature at 20—25°C; 56.0 g (0.494 moles) of 30% aqueous hydrogen peroxide is added in 30 minutes while maintaining the temperature at 20—30°C. The solution is heated to 80°C and stirred at 80—90°C until the reaction is complete as determined by thin layer chromatography (approximately two hours). After the reaction mixture is cooled to 20—30°C, 125 mL of methylene chloride is added and then 19.2 g (0.188 mol) of 96% sulfuric acid is added to neutralize the mixture. The organic layer is separated and the solvent is distilled off to give 31.7 g of 76.7% pure (76.0% yield) of solid 2-(5-isopropyl-5-methyl-4-oxo-2-imidazolin-2-yl)-*p*-toluic acid and 6-(5-isopropyl-5-methyl-4-oxo-2-imidazolin-2-yl)-*m*-toluic acid.

Example 8

Preparation of 2-(5-isopropyl-5-methyl-4-oxo-2-imidazolinyl-2-yl)-*p*-toluic acid and 6-(5-isopropyl-5-methyl-4-oxo-2-imidazolin-2-yl-*m*-toluic acid

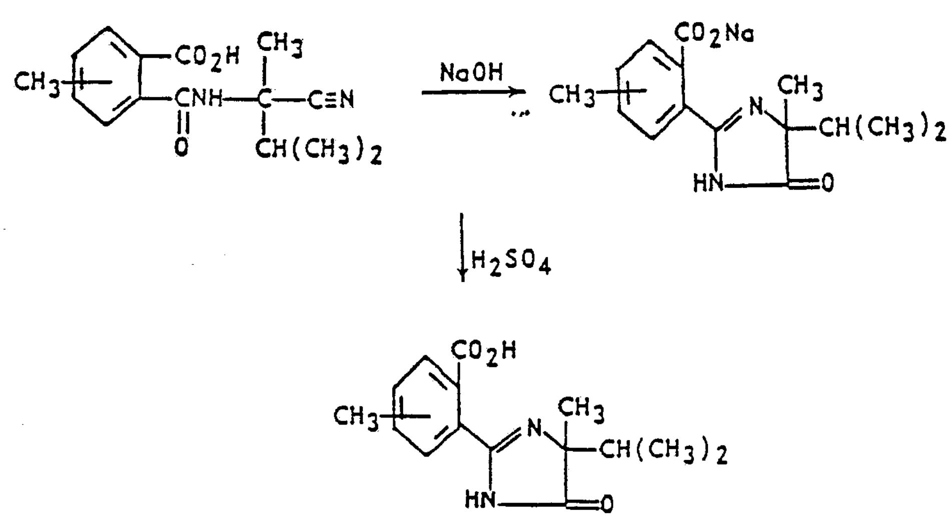

To 29.5 g (0.108 mol) of N-(1-cyano-1,2-dimethylpropyl)-4(and 5)-methyl phthalamic acid in 111 mL of water is added 26.4 g (0.33 mol) of aqueous 50% sodium hydroxide. External cooling is applied to hold the temperature at 20—25°C. The mixture is stirred for one hour at 20—25°C then heated to 80—90°C and stirred until the reaction is complete as determined by thin layer chromatography analysis of reaction mixture (approximately seven hours). The reaction mixture is cooled to 20—30°C and 125 mL of methylene chloride and 16.8 g (0.165 mol) of 96% sulfuric acid are added to neutralize the mixture. The organic layer is separated and the solvent removed *in vacuo* to give 26.3 g of 69.3% pure, (61.5% yield) solid 2-(5-isopropyl-5-methyl-4-oxo-2-imidazolin-2-yl)-*p*-toluic acid and 6-(5-isopropyl-5-methyl-4-oxo-2-imidazolin-2-yl-*m*-toluic acid.

Example 9

Preparation of 2-(5-isopropyl-5-methyl-4-oxo-2-imidazolin-2-yl)benzoic acid, hydrochloric salt

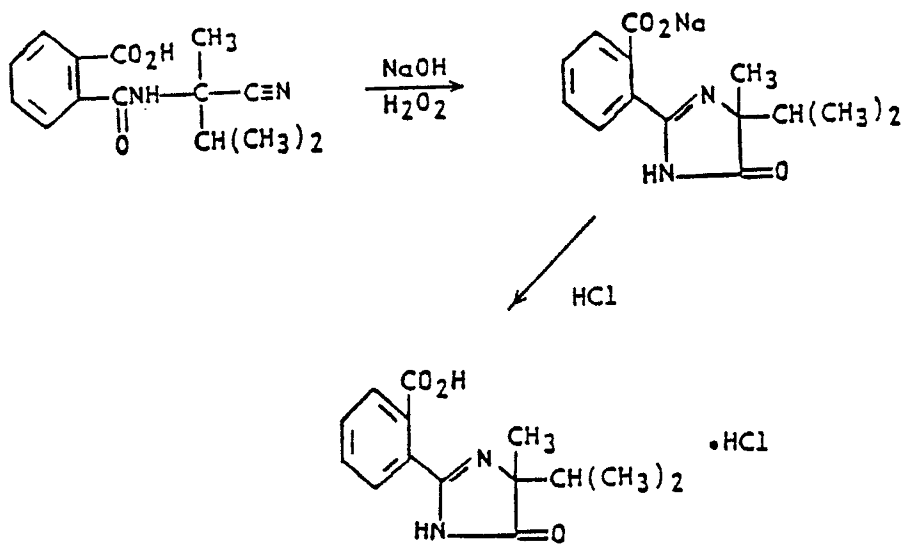


To 5.22 g (0.020 mol) of N-(1-cyano-1,2-dimethylpropyl)phthalamic acid in a mixture of 35 mL isopropyl alcohol and 10 mL of water is added 6.41 g (0.080 mol) of aqueous 50% sodium hydroxide. External cooling is applied to hold the temperature at 20—30°C and 4.80 g (0.042 mol) of aqueous 30% hydrogen peroxide is added. The mixture is heated at 80°C for five hours then cooled to 20—25°C. Aqueous 36% hydrochloric acid is added to adjust the pH to 1. The volatile solvent is removed *in vacuo* to give 8.35 g of solid 2-(5-isopropyl-5-methyl-4-oxo-2-imidazolin-2-yl)benzoic acid, hydrochloric salt. The solid is analyzed by high performance liquid chromatography. The real yield is 60.3%.

Example 10

Preparation of 2-(5-isopropyl-5-methyl-4-oxo-2-imidazolin-2-yl)nicotinic acid

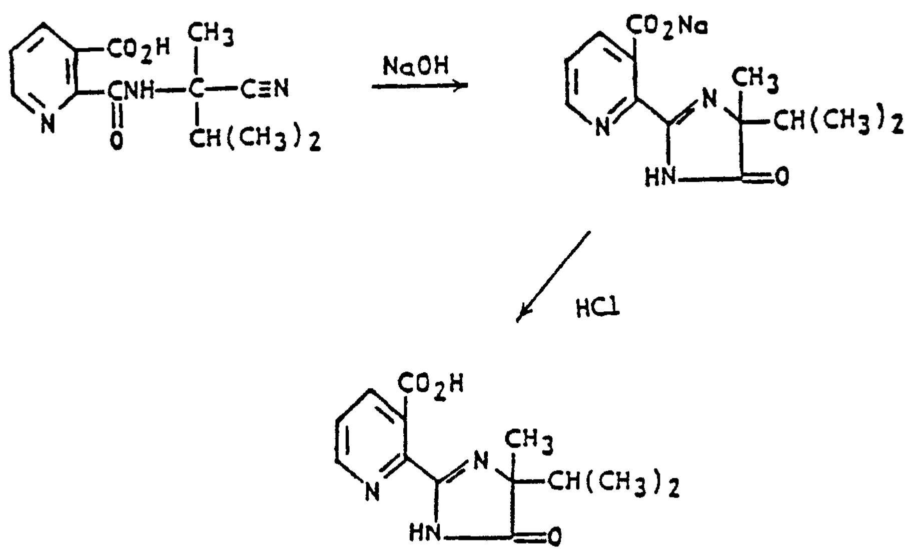

To a stirred mixture of 20 mL of water and 8.40 g (0.105 mol) of 50% sodium hydroxide solution is added 7.83 g (0.030 mol) of 2-[(1-cyano-1,2-dimethylpropyl)aminocarbonyl]nicotinic acid. External heating is applied to raise the temperature to 70—75°C where it is maintained for five hours. After cooling the reaction mixture to 20—30°C, 50 mL of methylene chloride is added and the pH is adjusted to 3.0 by the addition of 37% hydrochloric acid. The organic layer is separated and the solvent is removed by distillation to give 6.71 g of solid 2-(5-isopropyl-5-methyl-4-oxo-2-imidazolin-2-yl)nicotinic acid which is 90% pure in 77% yields.

Example 11

Preparation of 2-(5-isopropyl-5-methyl-4-oxo-2-imidazolin-2-yl)nicotinic acid

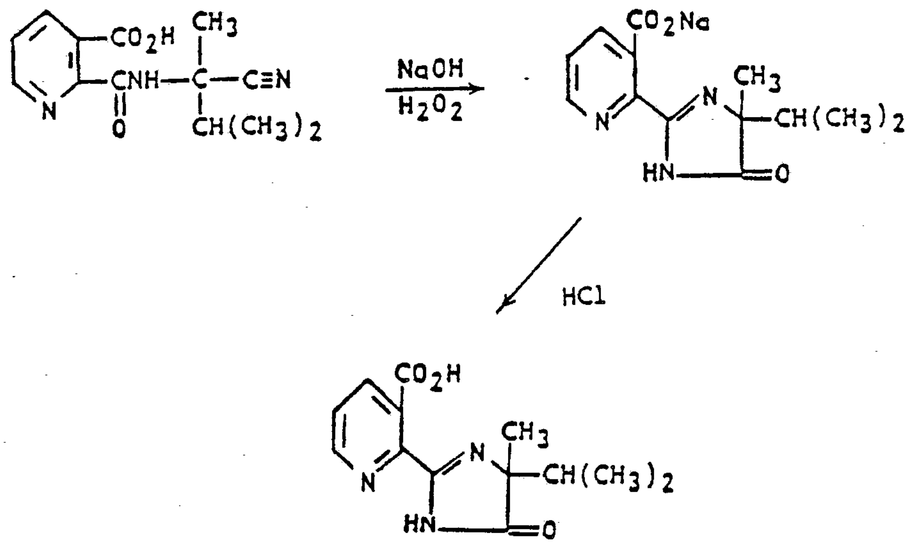


To a stirred mixture of 20 mL of water and 8.40 g (0.105 mol) of 50% sodium hydroxide solution is added 7.83 g (0.030 mol) of 2-[(1-cyano-1,2-dimethylpropyl)aminocarbonyl]-3-pyridinecarboxylic acid. External heating is applied to raise the temperature to 35—40°C. To this solution 13.6 g (0.120 mol) of 30% hydrogen peroxide is added over 30 minutes while maintaining the temperature at 35—40°C with external cooling. After one and one-half hours at 35—40°C, the mixture is heated to 70°C and is held at that temperature for two hours. After cooling to 20—30°C, 50 mL of methylene chloride is added and the pH is adjusted to 3.0 by the addition of 37% hydrochloric acid. The organic layer is separated and the solvent distilled off to give 7.57 g (87% yield) of solid 2-(5-isopropyl-5-methyl-4-oxo-2-imidazolin-2-yl)nicotinic acid.

Example 12

Preparation of 2-(5-isopropyl-5-methyl-4-oxo-imidazolin-2-yl)-3-quinolinecarboxylic acid

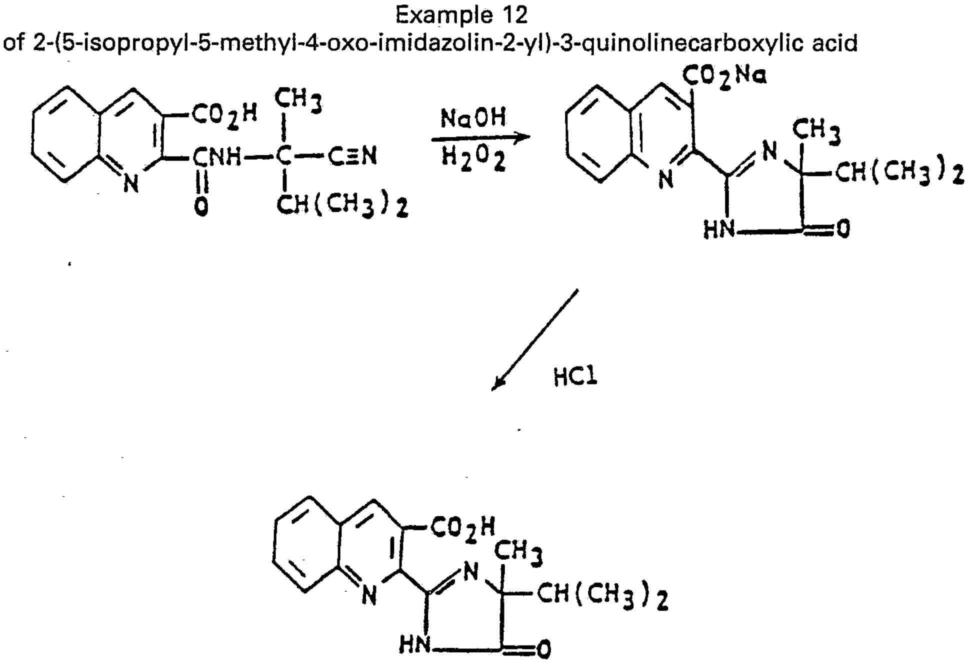

To a stirred solution of 1.69 g (0.042 mol) of solid sodium hydroxide in 11 mL of water is added 4.00 g (0.013 mol) of 2-[(1-cyano-1,2-dimethylpropyl)aminocarbonyl]-3-quinolinecarboxylic acid. External heating is applied to raise the temperature to 80—83°C. To this solution 4.37 g (0.039 mol) of 30% hydrogen peroxide is added over 30 minutes while maintaining the temperature at 80—83°C. After the hydrogen peroxide addition is complete, 1.04 g (0.026 mol) of solid sodium hydroxide is added. An additional 1.04 g (0.026 mol) of solid sodium hydroxide is added after 1 hour. Following a total reaction time of 2 hours, the solution is cooled in an ice-water bath and the pH is adjusted to 2.0 by the addition of 37% hydrochloric acid. The precipitate is filtered, washed, and dried to give 3.90 g (85% yield of solid 2-(5-isopropyl-5-methyl-4-oxo-2-imidazolin-2-yl)-3-quinolinecarboxylic acid which is 87.5% pure.

Example 13

Effect of base stoichiometry and concentration on the formation of 2-(5,5-disubstituted-4-oxo-2-imidazolin-2-yl)compounds

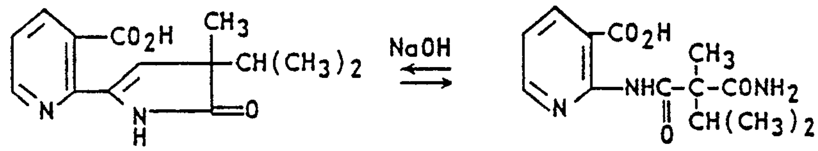


Three samples of 2-(5-isopropyl-5-methyl-4-oxo-2-imidazolin-2-yl)nicotinic acid (1.3 g, 0.005 mol) are dissolved in three 30% aqueous basic solutions containing, two, three and four molar equivalents of sodium hydroxide. Each of these solutions is heated at 60°C for three hours and the solution analyzed by high performance liquid chromatography for equilibrium concentrations of 2-(5-isopropyl-5-methyl-4-oxo-2-imidazolin-2-yl)nicotinic acid and 2-[(1-carbamoyl-1,2-dimethylpropyl)carbamoyl]nicotinic acid. Sufficient water is then added to each solution to adjust the base concentration to 15% and the equilibrium concentrations determined as above. Finally the base concentration is adjusted to 10% and the equilibrium concentrations determined. The results of these experiments illustrated graphically below demonstrate the importance of sufficient base concentration and stoichiometry for the efficient formation of the (2-imidazolin-2-yl) compounds.

Effect of base concentration and stoichiometry on the formation of 2-(5,5-disubstituted-4-oxo-2-imidazolin-2-yl)compounds

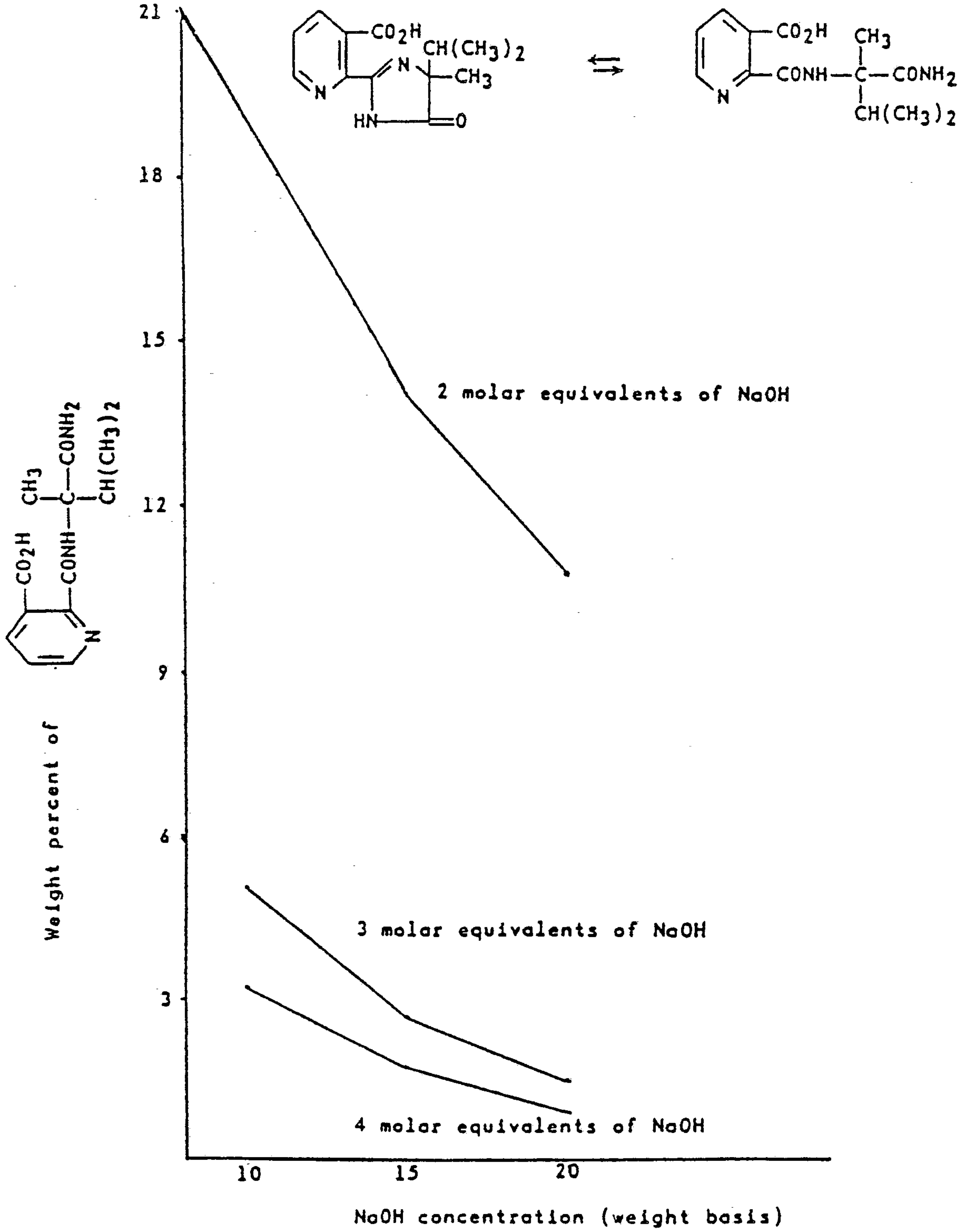
21
18
15
12
9
6
3
2 molar equivalents of NaOH
3 molar equivalents of NaOH
4 molar equivalents of NaOH
Weight percent of
10
15
20
NaOH concentration (weight basis)

Example 14

Preparation of 2-(5-isopropyl-5-methyl-4-oxo-2-imidazolin-2-yl)-*p*-toluic acid and 6-(5-isopropyl-5-methyl-4-oxo-2-imidazolin-2-yl)-*m*-toluic acid

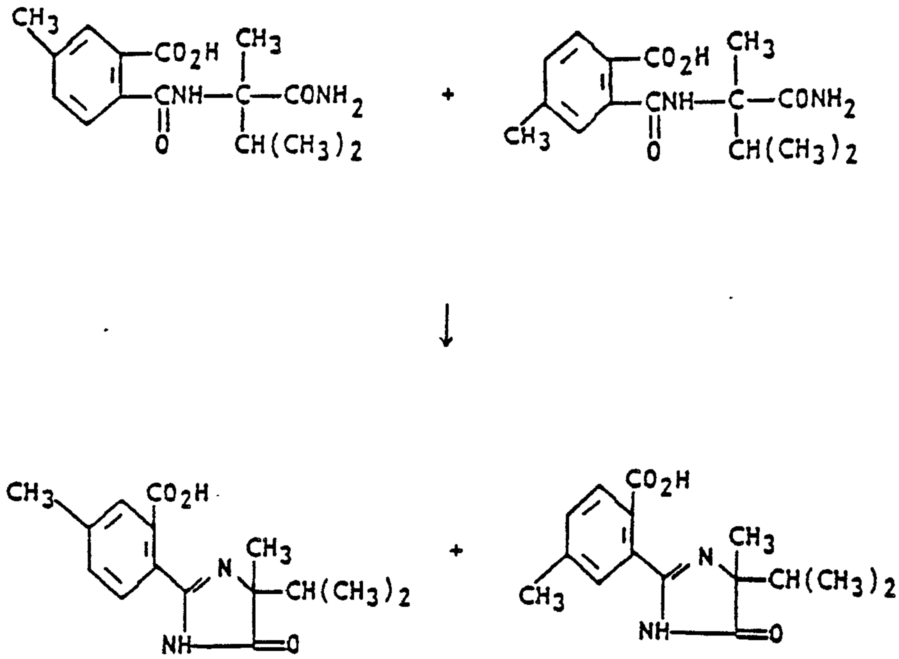

A mixture of the [(1-carbamoyl-1,2-dimethylpropyl)carbamoyl] *meta* and *para* toluic acids (5.83 g, 0.02 mol) is dissolved in 15% aqueous sodium hydroxide (0.4 mol, 20 molar equivalents). The solution is heated at 80°C for two hours then cooled to room temperature. Analysis of the reaction mixture by high performance liquid chromatography shows 94% of the desired mixture of 2-(5-isopropyl-5-methyl-4-oxo-2-imidazolin-2-yl)-*p*-toluic acid and 6-(5-isopropyl-5-methyl-4-oxo-imidazolin-2-yl)-*m*-toluic acid.

Example 15

Effect of base concentration on the formation of 2-(5,5-disubstituted-4-oxo-2-imidazolin-2-yl) compounds

Samples of 2-[(1-carbamoyl-1,2-dimethylpropyl)carbamoyl]nicotinic acid, *meta* and *para*-toluic acids are dissolved in 3.42 molar equivalents of aqueous sodium hydroxide at varying concentrations of 4%, 10%, and 20%. The resulting solutions are heated at 80 to 85°C for two to three hours and the solutions assayed for the desired cyclized (imidazolin-2-yl) products. The results of these experiments are reported in Table III below, which demonstrates a significant increase in product formation at base concentrations of 10% or greater on weight basis.

TABLE I

Effect of Base Concentration of the Formation of (Imidazolin-2-yl) Compounds of the Invention

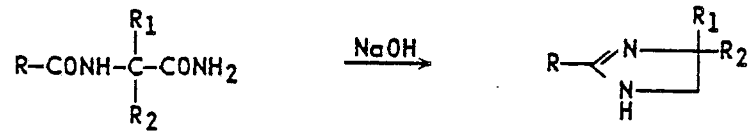


| R | NaOH Concentration | Time hrs | % yield |
|---|---|---|---|
| 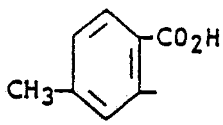 | 4 | 0.083 | 17.7 |
| + | 4 | 3.0 | 81.8 |
| 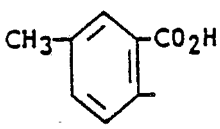 | 10 | 3.0 | 94.0 |
| 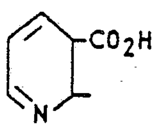 | 4 | 3.0 | 87.4 |
| | 10 | 3.0 | 96.5 |
| | 20 | 3.0 | 98.1 |

## Claims

1. A process for the preparation of a 2-(5,5-disubstituted-4-oxo(or thiono)-2-imidazolin-2-yl)nicotinic acid, 3-quinolinecarboxylic acid or benzoic acid of the formula:

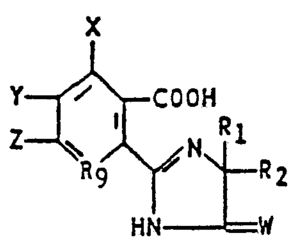

(IA)

wherein $R_9$ is N or CH; $R_1$ is $C_1$—$C_4$ alkyl; $R_2$ is $C_1$—$C_4$ alkyl or $C_3$—$C_6$ cycloalkyl; and when $R_1$ and $R_2$ are taken together along with the carbon to which they are attached, they may represent $C_3$—$C_6$ cycloalkyl optionally substituted with methyl, and when $R_1$ and $R_2$ are not the same, the optical isomers thereof; W is O or S; X is hydrogen, or $C_1$—$C_4$ alkyl, Y is hydrogen, halogen, $C_1$—$C_4$ alkyl, $C_1$—$C_4$ alkoxy, trifluoromethyl, trichloromethyl, difluoromethoxy, diloweralkylamino, $C_1$—$C_4$ alkylthio, nitro, or phenyl or phenoxy optionally substituted with one $C_1$—$C_4$ alkyl, $C_1$—$C_4$ alkoxy or halogen; Z is hydrogen, $C_1$—$C_4$ alkyl, trifluoromethyl, trichloromethyl, phenyl or phenyl substituted with one $C_1$—$C_4$ alkyl, $C_1$—$C_4$ alkoxy or halogen; and when taken together, Y and Z may form a ring in which YZ are represented by the structure: $—(CH_2)_n—$, where n is an integer from 3 to 5, provided that X is hydrogen; or

YZ is 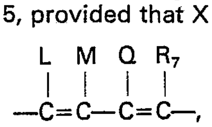,

where L, M, Q and $R_7$ are each of hydrogen, halogen, $C_{1-4}$ alkyl, $C_{1-4}$ alkoxy, $C_1$—$C_4$ haloalkyl, difluoromethoxy, diloweralkylamino, $C_1$—$C_4$ alkylthio, nitro, phenyl, phenoxy or mono-substituted phenyl or phenoxy where the substituent is $C_{1-4}$ alkyl, $C_1$—$C_4$ alkoxy or halogen; with the proviso that only one of L, M, Q or $R_7$, may represent a substituent other than hydrogen, halogen, $C_1$—$C_4$ alkyl or $C_1$—$C_4$ alkoxy; comprising, reacting a compound of the structure:

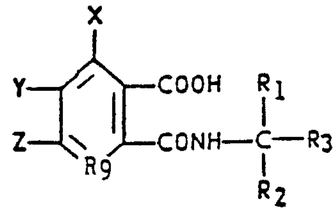

wherein $R_9$, X, Y, Z, W, $R_1$ and $R_2$ are as described above, and

$R_3$ is $\overset{W}{\overset{\|}{C}}$—$NH_2$ or CN,

with from 2 to 20 molar equivalents of an aqueous or aqueous alcoholic ($C_1$—$C_4$) sodium or potassium hydroxide of a concentration of 10% or greater and from 0 to 10 molar equivalents of 30 to 90% aqueous hydrogen peroxide at a temperature of from 25 to 110°C and thereafter acidifying the thus-formed reaction mixture to a pH between 2 and 4 with a strong mineral acid to give the formula (IA) acid.

2. A method according to Claim 1, wherein the base concentration is from 10 to 40% of the total reaction mixture on a weight basis, used in sufficient quantities to provide from two to six molar equivalents of base for each equivalent of formula (Ia) product.

3. A method according to claim 1 for the preparation of a formula (IA), 2-(5,5-disubstituted-4-oxo(or thiono)-2-imidazolin-2-yl)nicotinic acid, 3-quinolinecarboxylic acid or benzoic acid; comprising, reacting a mixture of compounds having the structures:

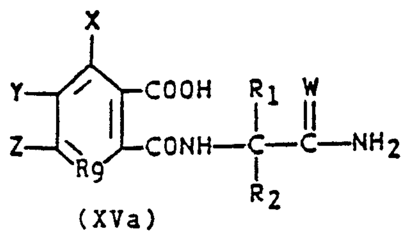

(XVa)

+

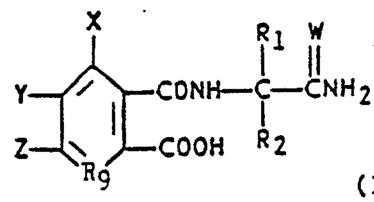

(XVb)

wherein $R_1$, $R_2$, $R_9$, X, Y, Z and W are as defined in claim 1 with 2 to 20 moles of an aqueous or aqueous $C_1$—$C_4$ alcoholic solution of sodium or potassium hydroxide of a concentration of 10% or greater per mole of formula (XVa) and (XVb) compound, at a temperature of 25 to 110°C, acidifying the thus-formed reaction mixture to a pH between 2 and 4 with hydrochloric acid or sulfuric acid, extracting the acidified reaction mixture with an organic solvent and separating the solvent from reaction mixture to obtain the formula (IA) acid product.

4. A method according to Claim 1 for the preparation of a compound having the structure (IA):

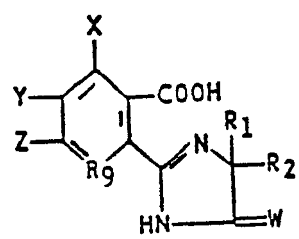

(IA)

where $R_9$, X, Y, Z, W, $R_1$ and $R_2$ are as described in Claim 1, comprising, reacting a compound of the formula:

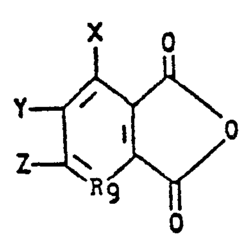

(XVI)

wherein $R_9$, X, Y, and Z are as described in Claim 1, with an equivalent amount of a compound of the formula:

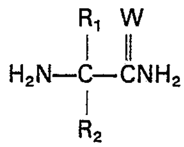

wherein $R_1$, $R_2$ and W are as described in said Claim 1, in the presence of a solvent of diethyl ether, tetrahydrofuran, dimethoxyethane, acetonitrile, or a halogenated hydrocarbon, at a temperature between 20 and 60°C under a blanket of nitrogen, to obtain an isomeric mixture of the compounds of formula (XVa) having the structure:

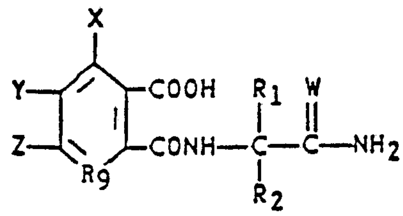

(XVa)

and formula (XVb) having the structure:

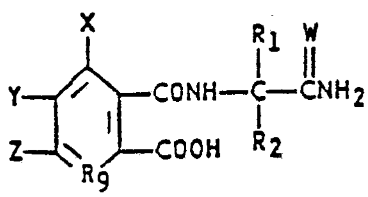

(XVb)

wherein $R_9$, X, Y, Z, W, $R_1$ and $R_2$ are as described in Claim 1, treating the thus formed reaction product with 2 to 10 moles of aqueous or aqueous $C_1$—$C_4$ alcoholic sodium or potassium hydroxide of a concentration of 10% or greater per mole of formula (XVa) and (XVb) compound, at a temperature of 25 to 110°C, acidifying the thus-formed reaction mixture to a pH between 2 and 4 with hydrochloric acid or sulfuric acid, extracting the acidified reaction mixture with an organic solvent and separating the solvent from the formula (IA) acid product.

5. A method according to Claim 1 for the preparation of a compound having the structure (IB):

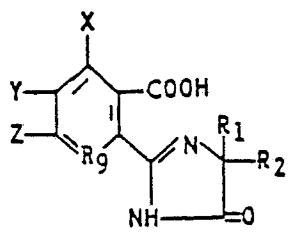

(IB)

wherein $R_9$, X, Y, Z, $R_1$ and $R_2$ are as described in Claim 1, comprising, reacting a compound of the formula:

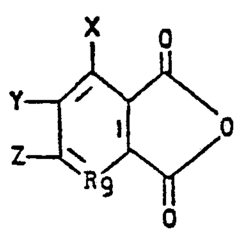

(XVI)

wherein $R_9$, X, Y and Z are as described in Claim 1, with an equivalent amount of a compound of the formula:

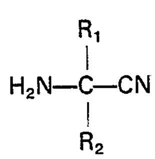

wherein $R_1$ and $R_2$ are as described in said Claim 1, in the presence of a solvent of diethyl ether, tetrahydrofuran, dimethoxyethane, acetonitrile or a low-boiling halogenated hydrocarbon, at a temperature between 20 and 60°C under a blanket of nitrogen, to obtain an isomeric mixture of the compounds having the structures (a) and (b):

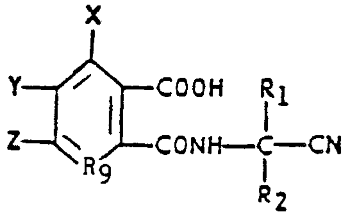

and

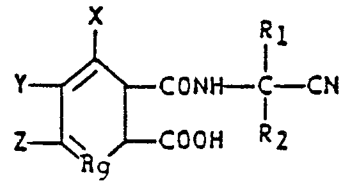

(a) (b)

wherein A, X, Y, Z, $R_1$ and $R_2$ are as described in Claim 1, treating the thus-formed reaction product with 2 to 10 moles of aqueous or aqueous $C_1$—$C_4$ alcoholic sodium or potassium hydroxide of a concentration of 10% or greater and 2 to 5 moles of 30 to 90% aqueous hydrogen peroxide per mole of formula (a) and (b) compound, at a temperature of 25 to 110°C, acidifying the thus-formed reaction mixture to a pH between 2 and 4 with hydrochloric acid or sulfuric acid, extracting the acidified reaction mixture with an organic solvent and separating the solvent from the formula (IB) acid product.

6. A method according to Claim 1 wherein $R_1$ is methyl; $R_2$ is isopropyl; W is O; X is hydrogen; Y is hydrogen, halogen, $C_1$—$C_4$ alkyl, $C_1$—$C_4$ alkoxy, trifluoromethyl, trichloromethyl, difluoromethoxy, diloweralkylamino, $C_1$—$C_4$ alkylthio, nitro, phenyl, phenoxy, or phenyl or phenoxy substituted with one $C_1$—$C_4$ alkyl, $C_1$—$C_4$ alkoxy or halogen and Z represents hydrogen, $C_1$—$C_4$ alkyl, trifluoromethyl, trichloromethyl, phenyl or phenyl substituted with one $C_1$—$C_4$ alkyl, $C_1$—$C_4$ alkoxy or halogen.

7. A method according to Claim 1 for the preparation of (+)-2-(5-isopropyl-5-methyl-4-oxo-2-imidazolin-2-yl)-3-quinolinecarboxylic acid.

8. A method according to Claim 1 for the preparation of (+)-2-(5-isopropyl-5-methyl-4-oxo-2-imidazolin-2-yl)nicotinic acid.

9. A method according to Claim 1 for the preparation of the mixture of compounds 2-(5-isopropyl-5-methyl-4-oxo-2-imidazolinyl-2-yl)-*p*-toluic acid and 6-(5-isopropyl-5-methyl-4-oxo-2-imidazolin-2-yl)-*m*-toluic acid.

**Patentansprüche**

1. Verfahren zur Herstellung einer 2-(5,5-Disubst.-4-oxo(oder thiono)-2-imidazolin-2-yl)-nikotinsäure, -3-chinolincarbonsäure oder -benzosäure der Formel:

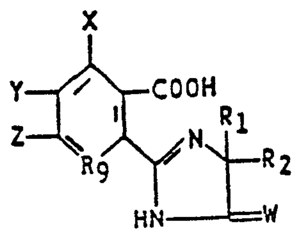

(IA)

wobei $R_9$ für N oder CH steht; $R_1$ für $C_1$—$C_4$-Alkyl steht; $R_2$ für $C_1$—$C_4$-Alkyl oder $C_3$—$C_6$-Cycloalkyl steht; und wobei $R_1$ und $R_2$, wenn sie zusammengefaßt sind, gemeinsam mit dem Kohlenstoff, an das sie geknüpft sind, $C_3$—$C_6$-Cycloalkyl, gegebenenfalls substituiert mit Methyl, bedeuten können, und falls $R_1$ und $R_2$ nicht gleich sind, die optischen Isomeren derselben; W für O oder S steht; X für Wasserstoff oder $C_1$—$C_4$-Alkyl steht; Y für Wasserstoff, Halogen, $C_1$—$C_4$-Alkyl, $C_1$—$C_4$-Alkoxy, Trifluormethyl, Trichlormethyl, Difluormethoxy, Di-niederalkylamino, $C_1$—$C_4$-Alkylthio, Nitro oder Phenyl oder Phenoxy, gegebenenfalls substituiert mit einem $C_1$—$C_4$-Alkyl, $C_1$—$C_4$-Alkoxy oder Halogen, steht; Z für Wasserstoff, $C_1$—$C_4$-Alkyl, Trifluormethyl, Trichlormethyl, Phenyl oder Phenyl, das substituiert ist mit einem $C_1$—$C_4$-Alkyl, $C_1$—$C_4$-Alkoxy oder Halogen, steht; und wobei Y und Z, wenn sie zusammengefaßt sind, einen Ring bilden können, in dem YZ für die Struktur $—(CH_2)_n—$ steht, wobei n eine ganze Zahl von 3 bis 5 ist, mit der Maßgabe, daß X Wasserstoff ist; oder wobei

YZ für 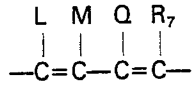 steht,

wobei L, M, Q und $R_7$ jeweils Wasserstoff, Halogen, $C_1$—$C_4$-Alkyl, $C_1$—$C_4$-Alkoxy, $C_1$—$C_4$-Haloalkyl, Difluormethoxy, Di-niederalkylamino, $C_1$—$C_4$-Alkylthio, Nitro, Phenyl, Phenoxy oder mono-substituiertes Phenyl oder Phenoxy bedeuten, wobei der Substituent $C_1$—$C_4$-Alkyl, $C_1$—$C_4$-Alkoxy oder Halogen ist; mit der Maßgabe, daß lediglich einer der Reste L, M, Q oder $R_7$ für einen Substituenten stehen kann, der nicht Wasserstoff, Halogen, $C_1$—$C_4$-Alkyl oder $C_1$—$C_4$-Alkoxy ist; umfassend die Umsetzung einer Verbindung der Struktur

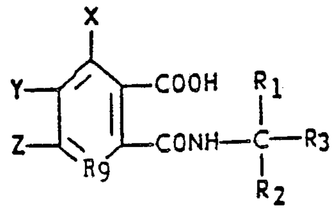

wobei $R_9$, X, Y, Z, W, $R_1$ und $R_2$ die oben angegebene Bedeutung haben und

$R_3$ für $\overset{W}{\overset{\|}{C}}—NH_2$ oder CN steht,

mit 2 bis 20 molaren Äquivalenten eines wässrigen oder wässrig-alkoholischen ($C_1$—$C_4$)-Natrium- oder Kaliumhydroxid mit einer Konzentration von 10% oder größer und von 0 bis 10 molaren Äquivalenten 30—90% wässrigem Wasserstoffperoxid bei einer Temperatur von 25 bis 110°C und anschließendes Ansäuern des so gebildeten Reaktionsgemisches auf einen pH zwischen 2 und 4 mit einer starken Mineralsäure, um die Säure der Formel (IA) zu erhalten.

2. Verfahren gemäß Anspruch 1, wobei die Basenkonzentration von 10 bis 40% der gesamten Reaktionsmischung auf Gewichtsbasis beträgt und in ausreichender Menge verwendet wird zur Schaffung von 2 bis 6 molaren Äquivalenten der Base für jedes Äquivalent des Produkts der Formel (IA).

3. Verfahren gemäß Anspruch 1 zur Herstellung einer 2-(5,5-Disubst.-4-oxo(oder thiono)-2-imidazolin-2-yl)nikotinsäure, -3-chinolincarbonsäure oder -benzosäure der Formel (IA), umfassend die Umsetzung einer Mischung von Verbindungen mit den Strukturen:

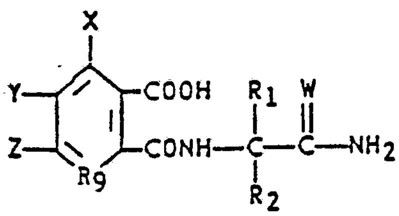

(XVa)

+

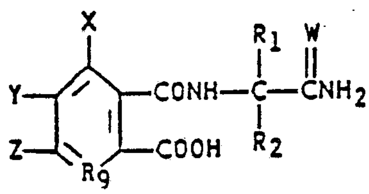

(XVb)

wobei $R_1$, $R_2$, $R_9$, X, Y, Z und W wie in Anspruch 1 definiert sind, mit 2 bis 20 Mol einer wässrigen oder wässrig $C_1$—$C_4$-alkoholischen Lösung von Natrium- oder Kaliumhydroxid mit einer Konzentration von 10% oder größer pro Mol der Verbindung der Formel (XVa) und (XVb), bei einer Temperatur von 25 bis 110°C, Ansäuern der so gebildeten Reaktionsmischung auf einen pH zwischen 2 und 4 mit Chlorwasserstoffsäure oder Schwefelsäure, Extrahieren der angesäuerten Reaktionsmischung mit einem organischen Lösungsmittel und Abtrennung des Lösungsmittels von der Reaktionsmischung, um das Säureprodukt der Formel (IA) zu erhalten.

4. Verfahren gemäß Anspruch 1 zur Herstellung einer Verbindung mit der Struktur (IA)

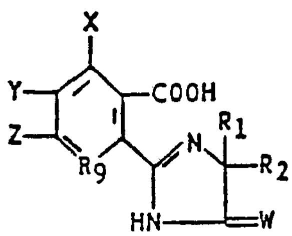

(IA)

wobei $R_9$, X, Y, Z, W, $R_1$ und $R_2$ wie in Anspruch 1 beschrieben sind, umfassend die Umsetzung einer Verbindung der Formel

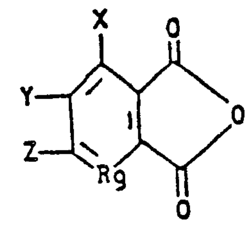

(XVI)

wobei $R_9$, X, Y und Z wie in Anspruch 1 beschrieben sind, mit einer äquivalenten Menge einer Verbindung der Formel

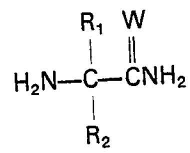

wobei $R_1$, $R_2$ und W wie in Anspruch 1 beschrieben sind, in Gegenwart eines Lösungsmittels von Diethylether, Tetrahydrofuran, Dimethoxyethan, Acetonitril oder einem halogenierten Kohlenwasserstoff, bei einer Temperatur zwischen 20 und 60°C unter einer Stickstoffdecke, um ein isomeres Gemisch von Verbindungen der Formel (XVa) mit der folgenden Struktur

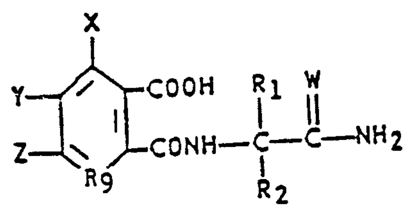

(XVa)

und der Formel (XVb) mit der folgenden Struktur

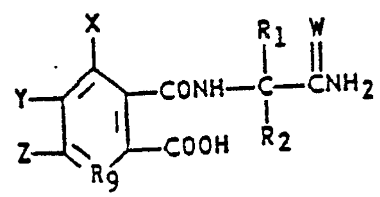

(XVb)

zu erhalten, wobei $R_9$, X, Y, Z, W, $R_1$ und $R_2$ wie in Anspruch 1 beschrieben sind, Behandlung des so gebildeten Reaktionsprodukts mit 2 bis 10 Mol wässrigem oder wässrig $C_1$—$C_4$-alkoholischem Natrium- oder Kaliumhydroxid mit einer Konzentration von 10% oder größer pro Mol der Verbindung der Formel (XVa) und (XVb), bei einer Temperatur von 25 bis 110°C, Ansäuern der so gebildeten Reaktionsmischung auf einen pH zwischen 2 und 4 mit Chlorwasserstoffsäure oder Schwefelsäure, Extrahieren der angesäuerten Reaktionsmischung mit einem organischen Lösungsmittel und Abtrennung des Lösungsmittels von dem Säureprodukt der Formel (IA).

5. Verfahren gemäß Anspruch 1 zur Herstellung einer Verbindung mit der Struktur (IB):

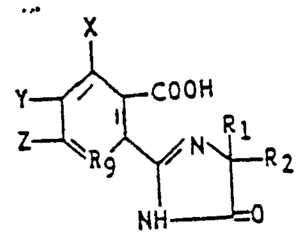

(IB)

wobei $R_9$, X, Y, Z, $R_1$ und $R_2$ wie in Anspruch 1 beschrieben sind, umfassend die Umsetzung einer Verbindung der Formel

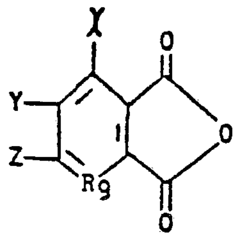

(XVI)

wobei $R_9$, X, Y und Z wie in Anspruch 1 beschrieben sind, mit einer äquivalenten Menge einer Verbindung der Formel

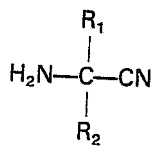

wobei $R_1$ und $R_2$ wie in Anspruch 1 beschrieben sind, in Anwesenheit eines Lösungsmittels von Diethylether, Tetrahydrofuran, Dimethoxyethan, Acetonitril oder einem niedrigsiedenden halogenierten Kohlenwasserstoff bei einer Temperatur zwischen 20 und 60°C unter einer Stickstoffdecke, um eine isomere Mischung von Verbindungen mit den Strukturen (a) und (b) zu erhalten:

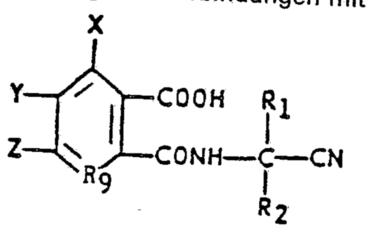

(a)

und

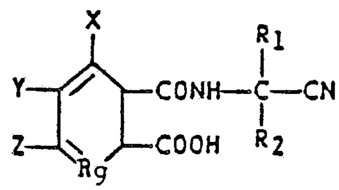

(b)

wobei A, X, Y, Z, $R_1$ und $R_2$ wie in Anspruch 1 beschrieben sind, Behandlung des so gebildeten Reaktionsprodukts mit 2 bis 10 Mol wässrigem oder wässrig $C_1$—$C_4$-alkoholischem Natrium- oder Kaliumhydroxid mit einer Konzentration von 10% oder größer und 2 bis 5 Mol 30 bis 90% wässrigem Wasserstoffperoxid pro Mol einer Verbindung der Formel (a) und (b), bei einer Temperatur von 25 bis 110°C, Ansäuern der so gebildeten Reaktionsmischung auf einen pH zwischen 2 und 4 mit Chlorwasserstoffsäure oder Schwefelsäure, Extrahieren der angesäuerten Reaktionsmischung mit einem organischen Lösungsmittel und Abtrennung des Lösungsmittels von dem Säureprodukt der Formel (IB).

6. Verfahren gemäß Anspruch 1, wobei $R_1$ für Methyl steht; $R_2$ für Isopropyl steht; W für O steht; X für Wasserstoff steht; Y für Wasserstoff, Halogen, $C_1$—$C_4$-Alkyl, $C_1$—$C_4$-Alkoxy, Trifluormethyl, Trichlormethyl, Difluormethoxy, Di-niederalkylamino, $C_1$—$C_4$-Alkylthio, Nitro, Phenyl, Phenoxy oder Phenyl oder Phenoxy, substituiert mit einem $C_1$—$C_4$-Alkyl, $C_1$—$C_4$-Alkoxy oder Halogen, steht; und Z für Wasserstoff, $C_1$—$C_4$-Alkyl, Trifluormethyl, Trichlormethyl, Phenyl oder Phenyl, substituiert mit einem $C_1$—$C_4$-Alkyl, $C_1$—$C_4$-Alkoxy oder Halogen, steht.

7. Verfahren gemäß Anspruch 1 zur Herstellung von (+)-2-(5-Isopropyl-5-methyl-4-oxo-2-imidazolin-2-yl)-3-chinolincarbonsäure.

8. Verfahren gemäß Anspruch 1 zur Herstellung von (+)-2-(5-Isopropyl-5-methyl-4-oxo-2-imidazolin-2-yl)nikotinsäure.

9. Verfahren gemäß Anspruch 1 zur Herstellung des Gemisches der Verbindungen 2-(5-Isopropyl-5-methyl-4-oxo-2-imidazolinyl-2-yl)-*p*-toluolsäure und 6-(5-Isopropyl-5-methyl-4-oxo-2-imidazolin-2-yl-*m*-toluolsäure.

**Revendications**

1. Procédé de préparation d'un acide 2-(5,5-disubstitué-4-oxo(ou thiono)-2-imidazolin-2-yl)nicotinique, 3-quinoléinecarboxylique ou benzoïque de formule:

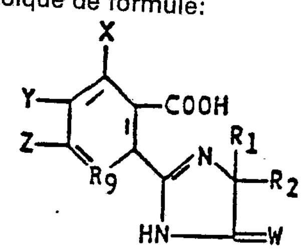

(IA)

où $R_9$ représente N ou CH; $R_1$ est un groupe alkyle en $C_1$—$C_4$, $R_2$ est un groupe alkyle en $C_1$—$C_4$ ou un groupe cycloalkyle en $C_3$—$C_6$; et lorsque $R_1$ et $R_4$ sont pris ensemble avec l'atome de carbone auquel ils sont liés, ils peuvent représenter un groupe cycloalkyle en $C_3$—$C_6$ éventuellement substitué avec un groupe méthyle et lorsque $R_1$ et $R_2$ sont différents, ses isomères optiques; W représente O ou S; X est un atome d'hydrogène ou un groupe alkyle en $C_1$—$C_4$, Y est un atome d'hydrogène, d'halogène, un groupe alkyle en $C_1$—$C_4$, un groupe alcoxy en $C_1$—$C_4$, un groupe trifluorométhyle, trichlorométhyle, difluorométhoxy, di(alkyl inférieur)amino, alkylthio en $C_1$—$C_4$, nitro, ou un groupe phényle ou phénoxy éventuellement substitué avec un groupe alkyle en $C_1$—$C_4$, alcoxy en $C_1$—$C_4$ ou un atome d'halogène; Z représente un atome d'hydrogène, un groupe alkyle en $C_1$—$C_4$, un groupe trifluorométhyle, trichlorométhyle, phényle ou phényle substitué avec un groupe alkyle en $C_1$—$C_4$, alcoxy en $C_1$—$C_4$ ou un atome d'halogène; et lorsqu'ils sont pris ensemble Y et Z peuvent former un cycle dans lequel YZ représente la structure: $-(CH_2)_n-$ où n est un entier choisi de 3 à 5 à condition que X soit un atome d'hydrogène, ou

YZ représente 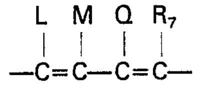

dans laquelle L, M, Q et $R_7$ représentent chacun un atome d'hydrogène, un atome d'halogène, un groupe alkyle en $C_1$—$C_4$, alcoxy en $C_1$—$C_4$, haloalkyle en $C_1$—$C_4$, difluorométhoxy, di(alkyl inférieur)amino, alkylthio en $C_1$—$C_4$, nitro, phényle, phénoxy, ou un groupe phényle ou phénoxy monosubstitué dans lequel le substituant est un groupe alkyle en $C_1$—$C_4$, alcoxy en $C_1$—$C_4$ ou un atome d'halogène à condition que seul l'un de L, M, Q ou $R_7$ représente un substituant autre qu'un atome d'hydrogène, un atome d'halogène, un groupe alkyle en $C_1$—$C_4$ ou alcoxy en $C_1$—$C_4$; dans lequel on fait réagir un composé de structure:

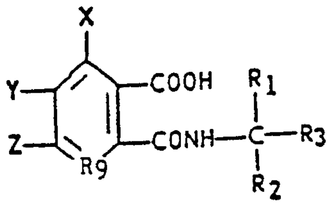

dans laquelle $R_9$, X, Y, Z, W, $R_1$ et $R_2$ sont tels que définis ci-dessus et

$R_3$ représente 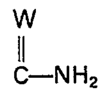 or CN

avec de 2 à 20 équivalents molaires d'une solution aqueuse ou aqueuse alcoolique($C_1$—$C_4$)d'hydroxyde de potassium ou de sodium en une concentration de 10% ou plus et avec de 0 à 10 équivalents molaires de peroxyde d'hydrogène aqueux à 30—90% à une température de 25 à 110°C et dans lequel on acidifie ensuite le mélange réactionnel ainsi formé jusqu'à un pH de 2 à 4 avec un acide minéral fort pour obtenir l'acide de formule (IA).

2. Procédé suivant la revendication 1, dans lequel la concentration en base est de 10 à 40% en poids par rapport au mélange réactionnel total, et est utilisée en une quantité suffisante pour fournir de 2 à 6 équivalents molaires de base pour chaque équivalent du produit de formule (IA).

3. Procédé suivant la revendication 1 pour la préparation d'un acide 2-(5,5-disubstitué-4-oxo(ou thiono)-2-imidazolin-2-yl)nicotinique, 3-quinoléinecarboxylique ou benzoïque de formule (IA), dans lequel on fait réagir un mélange de composés de structures:

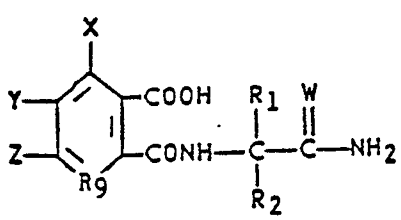

(XVa)

+

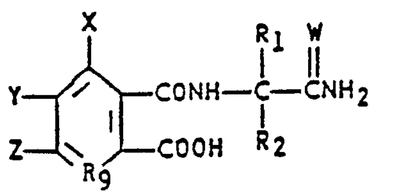

(XVb)

dans lesquelles $R_1$, $R_2$, $R_9$, X, Y, Z et W sont tels que définis dans la revendication 1 avec 2 à 20 moles d'une solution aqueuse ou aqueuse alcoolique(en $C_1$—$C_4$)d'hydroxyde de potassium ou de sodium en une concentration de 10% ou plus, par mole du composé de formule (XVa) et du composé de formule (XVb), à une température de 25 à 110°C, on acidifie le mélange réactionnel ainsi formé à un pH de 2 à 4 avec de l'acide chlorhydrique ou de l'acide sulfurique, et on extrait le mélange réactionnel acidifié avec un solvant organique et on sépare le solvant du mélange réactionnel pour obtenir le produit acide de formule (IA).

4. Procédé suivant la revendication 1 pour la préparation d'un composé ayant la structure (IA):

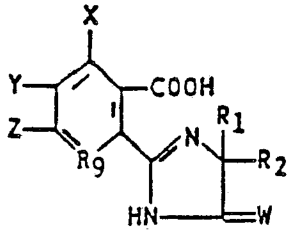

(IA)

dans laquelle $R_9$, X, Y, Z, W, $R_1$ et $R_2$ sont tels que définis dans la revendication 1, dans lequel on fait réagir un composé de formule:

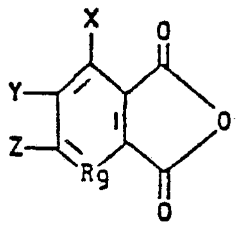

(XVI)

dans laquelle $R_9$, X, Y, et Z sont tels que définis dans la revendication 1 avec une quantité équivalente d'un composé de formule:

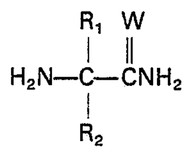

dans laquelle $R_1$, $R_2$ et W sont tels que définis dans la revendication 1, en présence d'un solvant constitué d'éther diéthylique, de tétrahydrofuranne, de diméthoxyéthane, d'acétonitrile ou d'un hydrocarbure halogéné, à une température de 20 à 60°C sous une atmosphère protectrice d'azote pour obtenir un mélange d'isomères des composés de formule (XVa) ayant la structure:

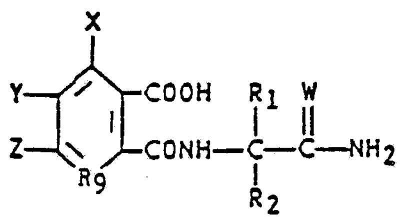

(XVa)

et de formule (XVb) ayant la structure:

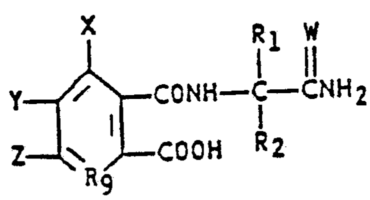

(XVb)

dans lesquelles $R_9$, X, Y, Z, W, $R_1$ et $R_2$ sont tels que définis dans la revendication 1, on traite le produit réactionnel ainsi formé avec de 2 à 10 moles d'une solution aqueuse ou aqueuse alcoolique(en $C_1$—$C_4$)d'hydroxyde de sodium ou de potassium en une concentration à 10% ou plus, par mole des composés de formule (XVa) et (XVb) à une température de 25 à 110°C, on acidifie le mélange réactionnel ainsi formé à un pH de 2 à 4 avec de l'acide chlorhydrique ou de l'acide sulfurique, on extrait le mélange réactionnel acidifié avec un solvant organique et on sépare le solvant du produit acide de formule (IA).

5. Procédé suivant la revendication 1 pour la préparation d'un composé de structure (IB):

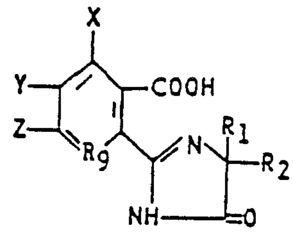

(IB)

dans laquelle $R_9$, X, Y, Z, $R_1$ et $R_2$ sont tels que définis dans la revendication 1, dans lequel on fait réagir un composé de formule:

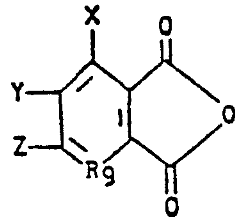

(XVI)

dans laquelle $R_9$, X, Y et Z sont tels que définis dans la revendication 1, avec une quantité équivalente d'un composé de formule:

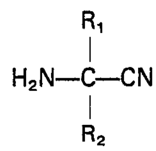

dans laquelle $R_1$ et $R_2$ sont tels que définis dans la revendication 1, en présence d'un solvant constitué d'éther diéthylique, de tétrahydrofuranne, de diméthoxyéthane, d'acétonitrile ou d'un hydrocarbure halogéné à bas point d'ébullition, à une température de 20 à 60°C, sous une atmosphère protectrice d'azote pour obtenir un mélange d'isomères des composés ayant les structures (a) et (b):

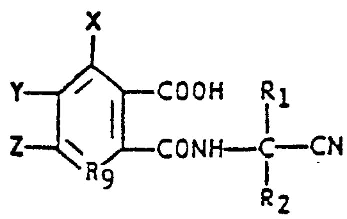

(a)

et

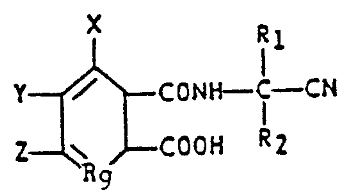

(b)

dans lesquelles $R_9$, X, Y, Z, $R_1$ et $R_2$ sont tels que définis dans la revendication 1, on traite le produit réactionnel ainsi formé avec 2 à 10 moles d'une solution aqueuse ou aqueuse alcoolique(en $C_1$—$C_4$)d'hydroxyde de sodium ou de potassium en une concentration de 10% au plus et avec de 2 à 5 moles de peroxyde d'hydrogène aqueux à 30—90% par mole des composés de formule (a) et (b) à une température de 25 à 110°C, on acidifie le mélange réactionnel ainsi formé jusqu'à un pH de 2 à 4 avec de l'acide chlorhydrique ou de l'acide sulfurique, on extrait le mélange réactionnel acidifié avec un solvant organique et on sépare le solvant du produit acide de formule (IB).

6. Procédé suivant la revendication 1, dans lequel $R_1$ est un groupe méthyle; $R_2$ est un groupe isopropyle; W représente O, X est un atome d'hydrogène; Y est un atome d'hydrogène, un atome d'halogène, un groupe alkyle en $C_1$—$C_4$, alcoxy en $C_1$—$C_4$, trifluorométhyle, trichlorométhyle, difluorométhoxy, di(alkyl inférieur)amino, alkylthio en $C_1$—$C_4$, nitro, phényle, phénoxy, ou un groupe phényle ou phénoxy substitué avec un groupe alkyle en $C_1$—$C_4$, alcoxy en $C_1$—$C_4$ ou un atome d'halogène, et Z représente un atome d'hydrogène, un groupe alkyle en $C_1$—$C_4$, trifluorométhyle, trichlorométhyle, un groupe phényle ou phényle substitué avec un groupe alkyle en $C_1$—$C_4$, alcoxy en $C_1$—$C_4$ ou un atome d'halogène.

7. Procédé suivant la revendication 1 pour la préparation de l'acide (+)-2-(5-isopropyl-5-méthyl-4-oxo-2-imidazolin-2-yl)-3-quinoléinecarboxylique.

8. Procédé suivant la revendication 1 pour la préparation de l'acide (+)2-(5-isopropyl-5-méthyl-4-oxo-2-imidazolin-2-yl)nicotinique.

9. Procédé suivant la revendication 1 pour la préparation du mélange d'acide 2-(5-isopropyl-5-méthyl-4-oxo-2-imidazolin-2-yl)-p-toluique et d'acide 6-(5-isopropyl-5-méthyl-4-oxo-2-imidazolin-2-yl)-m-toluique.